(19) Europäisches Patentamt
European Patent Office
Office européen des brevets

(11) Publication number: **0 047 781**
**B1**

(12) **EUROPEAN PATENT SPECIFICATION**

(45) Date of publication of patent specification: **02.01.85**

(51) Int. Cl.⁴: **G 03 C 7/30,** G 03 C 5/30, G 03 C 5/54

(21) Application number: **81900907.7**

(22) Date of filing: **16.03.81**

(86) International application number: **PCT/US81/00331**

(87) International publication number: **WO 81/02797 01.10.81 Gazette 81/23**

# (54) DYE-FORMING DEVELOPERS.

(30) Priority: **17.03.80 GB 8008984**

(43) Date of publication of application: **24.03.82 Bulletin 82/12**

(45) Publication of the grant of the patent: **02.01.85 Bulletin 85/01**

(84) Designated Contracting States: **CH DE FR GB LI SE**

(56) References cited:
**DE-A-2 727 090**
**GB-A-1 122 085**
**GB-A-2 017 951**
**US-A-3 491 151**
**US-A-3 622 629**
**US-A-4 126 461**

(73) Proprietor: **MINNESOTA MINING AND MANUFACTURING COMPANY**
**3M Center, P.O. Box 33427**
**St. Paul, MN 55133 (US)**

(72) Inventor: **SABONGI, Gabron J.**
**235 Barn Mead**
**Harlow, Essex (GB)**
Inventor: **VAN THIEN, Than**
**85 Moor Tower**
**Harlow, Essex (GB)**

(74) Representative: **Bowman, Paul Alan et al**
**LLOYD WISE, TREGEAR & CO. Norman House**
**105-109 Strand**
**London WC2R OAE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 047 781 B1

Courier Press, Leamington Spa, England.

**Description**

This invention relates to the production of black and white or color photographic images and in particular to photographic elements and to developer solutions containing dye-forming developers to amplify the silver image.

Metallic silver has been used overwhelmingly in the past to provide image density to most black and white photographic materials. Such an image is obtained through a process involving imagewise exposure of a silver salt light-sensitive material, usually silver halide, followed by a development step in which the exposed silver halide latent image is selectively reduced to metallic silver and finally a fixation step in which the unexposed silver salt is removed and the image is stabilized against further exposure. In contrast to black and white development in which developer does not form a dye image since the oxidised developer is discarded as a non-essential by-product, color development involving paraphenylene diamine type developers and color formers provides a dye image in combination with a silver image.

The growing scarcity and rising cost of silver has spurred interest in alternative light sensitive materials. However, none of these light sensitive materials has matched the sensitivity of silver halide. Numerous attempts have been made to amplify the silver image with dye-through color development using mostly cyan couplers, as disclosed for example in United States Patent Specification No. 4,126,461, British Patent Specification Nos. 1,524,017 and 1,530,398 and French Patent Specification No. 2,389,162.

An attractive method of achieving a dye amplified silver image consists of using a dye-forming developer which upon reducing the exposed silver halide to metallic silver, is itself transformed into a dye by subsequent oxidation or by dimerisation followed by further oxidation.

British Patent Specification No. 1,122,085 describes the use of 4-methoxy-1-naphthol and related compounds as developers for silver halide emulsions to provide a blue dye image known as Russig's Blue with the aim of reducing the silver consumption in radiographic materials. The disadvantage of using this alkaline developer formulation is its instability and susceptibility to air oxidation. The premature formation of the blue dye in the alkaline solution leads to a poor shelf life of the developer solution.

United States Patent Specification Nos. 3,057,712 and 3,148,060 disclose the use of 4-methoxy-1-naphthol incorporated in a non-tanning diffusing transfer developer for graphic arts. Again air oxidation is the main problem. United States Patent Specification Nos. 3,320,063 and 3,431,260 disclose the use of keto-1,3-perinaphthoxazines as silver halide developers which can form dyes for diffusion transfer processes.

British Patent Application No. 2,017,951A discloses the use of dihydroxynaphthalene derivatives as chromogenic developers, especially for radiographic materials. Various tints from brown to blue are reported. The drawback of such developers resides on the excessive solubility in alkali of the dyes which are easily washed out resulting in loss of density.

United States Patent Specification No. 2,206,126 discloses the use of monoethers of 1,2- and 1,4-dihydroxyanthracene along with a number of phenol, naphthol, hydroquinone derivatives and leuco indigoid dyes such as dye-forming developers, chiefly for color photography. So far these processes have not been put into practice due to the limited range of colors obtained, the instability of the developers, and the difficulty of preparing some of the developers.

The use of dye-forming developers has been hindered by their low stability in alkaline developer formulations and in the presence of air. For example, alkoxynaphthols oxidize and autocouple to form lignone dyes. British Patent Specification No. 1,122,085 discloses that 4-methoxy-1-naphthol, although being an adequate photographic developer, cannot be incorporated in a developer formulation containing sulfite since the latter adversely affects the dye density. This Patent Specification discloses the incorporation of 1-phenyl-3-pyrazolidone (phenidone) and exemplifies the use of a sulphite at 4 g/litre solution of developer.

The dihydroxynaphthalenes which are described in British Patent Specification No. 2,017,957 on reaction with the silver latent image provide dyes which are pH sensitive and have broad absorption ranges in the visible region giving rise to impure colors of a limited range. The specific developer solution disclosed comprises a dye-forming dihydroxynaphthalene compound in aqueous alkaline solution together with triethylene glycol. Furthermore hydroxylamine and ethanolamine may be added to the developer.

There remains a need for improved dye-forming developer compositions useful both for color and black and white photographic materials which can be conveniently developed by simple treatment with stabilized alkaline developer solutions.

According to one embodiment of the present invention there is provided a developer solution comprising an aqueous alkaline medium containing one or more dye-forming developing agents (as defined herein) having the general formula:

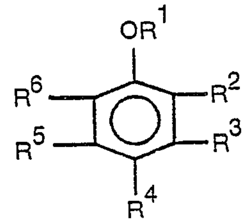

in which $R^1$ represents a hydrogen atom or hydrolysable group,

each of $R^2$ to $R^6$ is independently selected from a hydrogen or halogen atom, an alkyl, aryl, alkoxy, aryloxy or amino group each of which groups may be substituted, hydroxy group, a thiol group or a thioether group, or two or more adjacent groups from $R^2$ to $R^6$ may represent the necessary atoms to complete one or more carbocyclic or heterocyclic ring systems, and at least two of the following components:

(1) a water-miscible polar organic solvent,

(2) an alkanolamine, substituted alkanolamine, a monoamino carboxylic acid which may be substituted, or any combination thereof, and

(3) a hydroxylamine, substituted hydroxylamine or an aryl or alkyl hydroxamic acid which may be substituted, or any combination thereof.

According to a second embodiment of the invention there is provided a photographic development process in which an exposed silver halide photographic film is developed in an alkaline environment in the presence of one or more dye-forming developing agents (as defined herein) having the general formula:

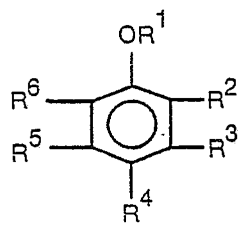

in which $R^1$ to $R^6$ are as defined above, and at least two of the following components

(1) a water-miscible polar organic solvent,

(2) an alkanolamine, substituted alkanolamine, a monoamino carboxylic acid which may be substituted, or any combination thereof, and

(3) a hydroxylamine, substituted hydroxyl-amine or an aryl or alkyl hydroxamic acid which may be substituted, or any combination thereof.

The term "dye-forming developing agent" used herein refers to a compound which under alkaline conditions in the presence of exposed silver halide will undergo a reaction involving oxidation of the compound to produce a dye which is substantive to gelatin. The dye forming developing agents used in the invention have the general formula. Generally at least one of the groups $R^2$, $R^4$ and $R^6$ of the formula (1) is a hydrogen atom or an oxidisable group, e.g., OH, NHR (in which R is hydrogen or alkyl) or

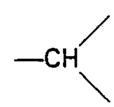 .

In the selection of groups $R^2$ to $R^6$, and generally in the specification for all R groups except where expressly defined differently, alkyl and alkoxy groups preferably have 1 to 30 carbon atoms, more preferably 1 to 12 carbon atoms and most preferably 1 to 3 carbon atoms. The longer carbon atom chains are particularly desirable when compatibility or solvency in long hydrocarbon chain organic solvents is desired as used in oil dispersed photographic emulsions. The aryl and aryloxy groups preferably have up to 30 carbon atoms, more preferably up to 15 carbon atoms, and most preferably are phenyl groups. The amino groups generally contain up to 30 carbon atoms (although each alkyl group therein may contain 30 carbon atoms), preferably contain 1 to 12 carbon atoms, and more preferably contain 2 to 6 carbon atoms (e.g., dimethyl, diethyl, and dipropyl amines). The alkyl groups for R are more preferably 1 to 12 carbon atoms and most preferably 1 to 4 carbon atoms.

Where the term group is used in describing substituents, photographically harmless substitution is anticipated on the substituent for example, alkyl group includes ether groups (e.g., $CH_3—CH_2—O—CH_2—$), haloalkyls, nitroalkyls, carboxyalkyls, hydroxyalkyls, etc. while the term alkyl includes only hydrocarbons. Substituents which react with active ingredients, such as with strong reducing substituents, would of course be excluded as not being photographically inert or harmless.

The invention allows the practical use of dye-forming developing agents to amplify the silver image in photographic films and formed colored images. Accordingly, it is possible to reduce the silver content of photographic films and obtain the same density. By a suitable choice of dye-forming developing agents a wide range of colored images may be obtained.

The developer solutions of the invention preferably contain all of components 1) to 3) and additionally include a black and white developing agent. it has been found that the above combination stabilizes the dye-forming developer (which is normally unstable to aerial oxidation in conventional alkaline photographic developer solutions) and thus allows the practical use of the developer to amplify the silver image in photographic films and form colored images.

A developer solution containing all of the components 1) to 3) and a black and white developing agent when tested using photographic films (e.g. microfilm, X-ray or graphic arts film) displayed:

a) a high stability against aerial oxidation over a period of about two weeks (without replenishment) at a working temperature of 28 to 30°C,

b) an image amplification through the increase in the image density (a total density of the dye and silver images),

c) these dyes displayed color purity and a range which included yellow, cyan and magenta colors; the bleaching of the silver metal image by bleaching agents known in the art of color photography, yielded colored images comparable to those obtained from the phenylene diamine-color coupler approach,

d) a process of forming an image inexpensively using a silver halide light-sensitive material, and

e) a pronounced matt surface finish to the photographic coating after processing in the above developer composition.

The black and white developing agents which may be used in the invention are well known and are disclosed, for example in *The theory of the Photographic Process,* Mees, James, 4th Edition, MacMillan 1977, Chapter II and references cited therein. The black and white developing agents are generally used in amounts up to 2 g-l of solution.

The dye-forming developing agents of formula (1) include naphthols, masked naphthols, bisphenols, bis-$\alpha$-naphthols, polynuclear hydroquinones and their monoethers, naphtho-hydroquinones and heterocyclic diols and will be described in detail hereinafter. The dye-forming developing agents may carry ballasting groups and/or solubilising groups which are known in the color photographic art. The dye-forming developing agents can be used alone or in combination to give the desired color and development rate.

The high stability of the developing solutions of the invention (as measured by the change in sensitometric D/log E curve characteristics and the absence of dye formation on standing in contact with air) is mainly due to the presence of two or more of the following components: alkanolamine, hydroxylamine and a water-miscible polar organic solvent.

The water-miscible polar organic solvents suitable for use in the developer solutions must not react with the other components in the solution under ambient and development conditions and should not be excessively volatile at the temperature at which the solutions are used for processing. The solvent component (1) is generally present to dissolve the dye-forming developing agent and is generally used in amounts up to 15% by weight of the developer solution, preferably 5 to 12% by weight. Suitable solvents include alkanols, e.g. methanol and isopropanol, and polyhydroxy organic solvents. A preferred polyhydroxy organic solvent is triethylene glycol (trigol) although other similar compounds such as simple and substituted glycols and polyalkoxy glycols may be used.

Compounds which can be used in place of hydroxylamine include substituted hydroxylamines and hydroxamic acids of the structure:

$$Z—CO—N(OH)—H \qquad (II)$$

in which Z represents a hydrogen atom, an alkyl or a substituted alkyl (preferably 1 to 8 carbon atoms), alkoxy, aryl, aryloxy, amino or substituted amino group all of which may have up to 30 carbon atoms. Hydroxamic acids are disclosed in United States Patent Specification No. 4,055,426. The hydroxylamine component (3) is generally present in the range of 1 to 15 g/l.

Compounds which may be used in place of alkanolamines are substituted alkanolamines, amino acids, amino benzoic acids and substitution derivatives of the above. Typical examples of alkanolamines which can be used in the process and compositions of the inventions include:

ethanolamine
diethanolamine
triethanolamine
di-isopropanolamine
2-methylaminoethanol
2-ethylaminoethanol

2-dimethylaminoethanol
2-diethylaminoethanol
1-diethylamino-2-propanol
3-diethylamino-1-propanol
3-dimethylamino-1-propanol
isopropylaminoethanol
2-amino-2-methyl-1,3-propanediol
ethylenediamine tetraisopropanol
benzyldiethanolamine
2-amino-2-(hydroxymethyl)-1,3-propanediol.

Suitable amino acids include aliphatic monoamino monocarboxylic acids of up to 3 carbon atoms, such as glycine, alanine and serine. Suitable aminobenzoic acids include ortho-aminobenzoic acid or para-amino-benzoic acid. The alkanolamine component (2) is generally used in an amount in the range 50 to 150 g/l.

The particular combination of components in the developer solution of the invention allows the content of sulfite, which is commonly used in black and white or dye-forming developer solutions, to be reduced or eliminated, thus avoiding or reducing the disadvantages resulting from the use of sulfite which attacks the dye or intermediates in the dye formation.

The developer solution may contain other known developer components. For example, an alkali, a buffering agent, e.g., sodium hydroxide, potassium hydroxide, sodium carbonate, potassium carbonate, sodium or potassium tertiary phosphate, potassium metaborate or borax may be used alone or in combination. Also in order to impart buffering action or raise the ionic strength of the developer, or for the convenience of preparing processing solution various salts such as disodium or dipotassium hydrogen phosphate, potassium or sodium dihydrogen phosphate, sodium or potassium bicarbonate, boric acid, alkali metal nitrates, and alkali metal sulfates may also be used.

For mechanical stability of coated emulsions, the developer solution can contain a hardener such as glutaraldehyde (10% aqueous solution) in amounts which range from 1 to 10 g, preferably about 5 g.

Furthermore, antifogging agents may be optionally incorporated into the developer solution, such as alkali metal halides and organic antifogging agents. Examples of organic antifogging agents are nitrogen-containing hetero-ring compounds, e.g., benzotriazole, 6-nitrobenzimidazole, 5-nitrobenzotriazole, 5-methylbenzotriazole and 5-nitrobenzotriazole, mercapto-substituted hetero-ring compounds, e.g., 1-phenyl-5-mercapto tetrazole, 2-mercaptobenzimidazole and 2-mercapto-benzothiazole, and mercapto-substituted aromatic compounds, e.g. thiosalicylic acid. The antifogging agents are generally used in an amount of about 1 mg to 5 g, preferably about 5 mg to 1 g per litre of developer solution.

Additional examples of nitrogen-containing hetero-ring compounds not substituted by a mercapto group are disclosed in the following literature: Nitrobenzimidazole compounds, British Patent Specification No. 403,789 and U.S. Patent Specification Nos. 2,496,940, 2,497,917 and 2,656,271. Benzotriazole compounds are disclosed in the Journal of Photographic Society of Japan, *11*, 48 (1948). Hetero-ring quaternary salts such as benzothiazolium salts are disclosed in United States Patent Specification Nos, 2,131,038, 1,694,716, 3,316,281. Tetrazaindene compounds are disclosed in United States Patent Specification Nos. 2,444,605, 2,444,606 and 2,447,607 and other hetero-ring compounds are disclosed in United States Patent Specification Nos. 2,173,628, 2,324,112 and 2,444,608. In addition, antifogging agents are disclosed in Kagaku Sashin Binran, Vol. 11, page 19 (Maruzen, 1959).

The developing solution may also include sodium sulfite, potassium sulfite, potassium bisulfite, or sodium bisulfite.

Optionally developer accelerators may be added to the developer solution if desired. Such development accelerators include cationic compounds such as various pyridinium compounds such as those described in United States Patent Specification No. 2,648,604, Japanese Patent Publication No. 9,503/69 and United States Patent Specification No. 3,671,247, cationic dyes such as phenosafranine, neutral salts such as thallium nitrate, potassium nitrate, non-ionic compounds such as polyethylene glycol or the derivatives thereof disclosed in Japanese Patent Publication No. 9,504/69, United States Patent Specification Nos. 2,533,990, 2,531,832, 2,950,970 and 2,577,127, and polythioethers, organic solvents described in L.F.A. Mason: *Photographic Processing Chemistry*, pp. 40 to 43 (Focal Press, London 1966).

Furthermore, benzyl alcohol and phenylethyl alcohol as disclosed in United States Patent Specification No. 2,515,147, pyridine as disclosed in Journal of Photographic Society of Japan, Vol. 14, page 74 (1952), ammonia, hydrazine and amines are effective development accelerators.

The developer solutions of the invention generally contain one or more dye-forming developing agents in an amount in the range 1 to 20 g/l of solution, preferably 7 to 12 g/l. A developer solution of the invention includes a mixture of cyan, magenta and yellow dye-forming developing agents which possess similar development rates which may be used to develop any black and white photographic materials to provide a substantially black dye amplified silver image.

The developer solutions of the invention generally have a pH above 9, preferably in the range 10.5 to 12. When a black and white developing agent (B.W.) is present, the amounts of dye-forming

developing agent (D.F.D.) and (B.W.) are generally selected so that the weight ratio (D.F.D.):(B.W.) is in the range 15:1 to 5:1, preferably about 10:1. When all components (1) to (3) are present, the weight ratio of solvent component (1):alkanolamine component (2):hydroxylamine component (3) is preferably about 15—8:10—3:3—0.1, and more preferably about 10:5:1 with each consecutive ingredient equal to or higher in weight proportion than the following ingredient.

The processing used in the present invention generally comprises a developing step and a fixing step and if necessary or desired, a water-washing step and a stopping step may be provided. A drying step may of course be provided after completing the processing.

The fixing solutions used can be acid or base fixing solutions known in the art of black and white or color photography.

In the case of acid fixing solutions, fixing at high temperatures, e.g. up to 35°C, and with the standard high levels of fixer components which are suitable for films with high silver content, can cause destruction of the dye image. Fixers which contain aluminum salts as gel hardeners, do show a more pronounced destruction of the dye density. To obviate this problem, since films having coatings of a lower silver content than standard ones may be used, the above standard fixer is generally utilized at higher dilution levels (4 to 6 times) than those recommended. At these dilution levels, fixers may be used safely at a range of temperatures between 20 to 35°C and at a variable fixing time of 30 to 60 seconds. Suitable fixers include standard commercial fixers, e.g. 3M Type microfilm fixer, 3M fixing solution, Kodak RP-Xomat fixer and May and Baker Ampfix.

Photographic coatings which are processed in a developer formulation similar to the above, are characterised by a significantly high imagewise matt surface finish which is specific for this developer formulation. Such a matt finish is useful in reducing light glare and reflection, especially for radiographic images.

The process of the invention also extends to a two or more bath development process in which the various components of the developer solution discussed above are separated into two or more solutions. When this technique is adopted, the dye-forming developing agent is generally present in a first bath having a pH insufficient to allow development, which pH generally reduces the developing agent's propensity to oxidise, and after immersion in the first bath the film is treated in a second bath having the remaining components and a high pH so taht activation takes place.

It also has been found that the dye-forming developing agents used in the invention may be incorporated in a photographic element to provide a dye amplified silver image upon development.

Therefore according to a further embodiment of the present invention there is provided a photographic element comprising a support having a photographic silver halide emulsion, the photographic element including a dye-forming developing agent (as defined herein) having the general formula:

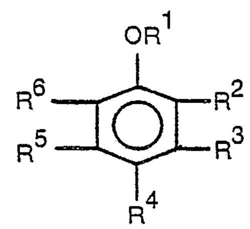

(1)

in which $R^1$ to $R^6$ are as defined above, in the silver halide emulsion layer, the dye-forming developing agent (as defined herein) being distributed in said layer(s) in an oil dispersion.

It has surprisingly been found that this particualr technique of incorporating the dye-forming developing agents in an oil dispersion in the layers of photographic elements provides excellent dye amplification of silver images which are generally superior to the results obtained when the dye-forming developing agents are incorporated into photographic elements by other methods. The oil dispersion technique provides a stable photographic element and is particularly useful for dye-forming developing agents which are water-insoluble or sparingly soluble in water. The dye-forming developing agents are distributed throughout the emulsion layer(s) dissolved in small droplets of solvents sometimes referred to in the art as oils. These usually have a particle size of about from 0.1 to 1.5, preferably 0.3 to 0.8 micrometers. An average droplet size of 0.4—0.6 and usually 0.5 micrometers is preferred.

The photographic element of the invention may be processed in a developer solution similar to that described of that of the invention but excluding the incorporation of a dye-forming developing agent in the solution. However, dye-forming developing agents may also be incorporated in the developer solution.

By a suitable selection of cyan, magenta and yellow dye-forming developing agents or precursors thereof which possess similar development rates and distributing them in a silver halide emulsion or in one or more layers adjacent said emulsion layer as described above, it is possible to provide a photographic element in which upon development the dyes will form a grey/black image. Following

imagewise exposure, such elements can be rapidly and conveniently developed to yield a substantially grey image merely by treatment with an alkaline activator solution so as to raise the pH in the element to a level which will either directly activate the incorporated developers or convert the developer precursors to their active form. The activated developers will simultaneously reduce the silver halide latent image to metallic silver and generate dyes imagewise.

If the coverage of silver halide in the element is sufficiently low so as not to give rise to appreciable background print-out during keeping, the developed photographic element can be simply fixed in an acidic stop-bath to stabilise the dye image and avoid loss of dye density. Otherwise a fixing step is required to remove the undeveloped halide using a bleach fix bath.

Any source of hydroxyl ions which will raise the pH of the element to an appropriate pH level (usually 11 to 14) is suitable. The optimum pH will depend on the particular dye-forming developers used. Developer precursors generally require a higher pH to be converted to their active form. The alkaline activator can be a simple aqueous solution of an inorganic base such as sodium silicate, sodium carbonate and sodium hydroxide, or contain additional development aids such as antifoggants, e.g. potassium bromide, 5-methylbenzotriazole, 4-carboxythiazoline-2-thione, and developing accelerators, e.g. an amine such as hydroxylamine, dimethylaminoethanol and triethanolamine or a quaternary ammonium salt, pyridinium compounds or a non-ionic surfactant, e.g. polyethylene glycol and derivatives thereof.

Alkaline activation can be performed at temperture and for a time sufficient to develop in the exposed element a visible image of acceptable density. Such results can be achieved by processing at times of several seconds to several minutes and at room temperature or at temperatures up to about 50°C. Whilst alkaline activation is sufficient to develop photographic elements of the invention, preferably the elements are developed in the presence of a black and white developing agent, present in either the emulsion or activation solution.

The storage stability of the photographic element of this invention can be improved by incorporating in the element such components as buffering agents, docating aids, hardeners, antifoggants, sensitising dyes, UV absorbers, and antihalation dyes. These formulations are well known to those skilled in the art of photographic silver halide emulsion.

Suitable emulsification processes suitable for the oil dispersion of the dye-forming developing agents are known in relation to the incorporation of color-couplers into photographic elements. Such techniques are disclosed for example in British Patent Specification Nos. 1,038,029, 1,052,487, 1,076,054, 1,077,426, 1,269,073 and 1,269,074 and in *Photographic Emulsion Chemistry,* G. F. Duffin, Focal Press 1966 and *Photographic Processing Chemistry,* L. F. A. Mason, Focal Press 1966, The developing agents can be dissolved in a high boiling and water immiscible organic solvent and vigorously stirred into the hydrophilic suspending medium with the help of an anionic surface active agent to create a stable dispersion of oily droplets. The high boiling solvents suitable for the present invention are well known to those skilled in the art of color photographic emulsions and include di-*n*-butyl phthalate, tri-*o*-cresyl-phosphate, N,N-diethyllauramide and ethylacetate.

There are a wide range of compounds having the general formula (1) which may be used as dye-forming developing agents in accordance with the invention. In the case of dye-forming developing agents in aqueous solution the group $R^1$ in formula (1) will be hydrogen. However, when the dye-forming developing agents are incorporated into the photographic element they may be present as a precursor of the agent which will form on contact with the alkaline developer solution. In such a case the group $R^1$ may be a hydrolysable group.

Naphthols suitable for use as dye-forming developing agents include alkoxy-1-naphthols, dialkylamino-1-naphthols and arylmethyl-1-naphthols.

Alkoxy-1-naphthols and masked naphthols include those of the general formula:

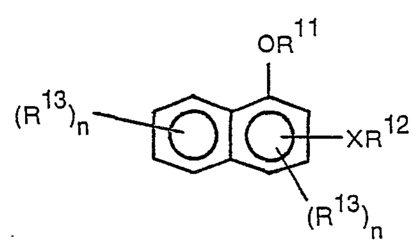

(2)

in which:

X is O, S or Se,

$XR^{12}$ can be in the 2 or 4 position,

$R^{11}$ is hydrogen or an alkali labile protecting group (i.e., a group which is converted to or replaced by hydrogen at a pH greater than 7.0), e.g. acetyl, chloroacetyl, dichloroacetyl, trichloroacetyl, trifluoroacetyl, carboalkyl, carboaryloxy, carbonate, benzoyl, *n*-nitrobenzoyl, 3,5-dinitrobenzoyl and 2-benzene-sulphonyl-1-ethoxycarbonyl,

$R^{12}$ represents a ballast group, e.g., alkyl, alkenyl, alkoxyalkyl, arylalkyl, aryloxyalkyl, alkylarylalkyl,

alkylaryloxyalkyl, alkylaryloxyalkyl, amino or dialkylaminoalkyl, trialkylammonium alkyl, acylamidoalkyl, carboxy and sulpho-containing alkyl, ester containing alkyl, these ballast groups are well known to those skilled in the art of silver halide photographic materials, and may contain up to 20 to 30 carbon atoms,

each $R^{13}$ independently represents a ring substituent selected among the following groups: hydrogen, alkyl, aryl, hydroxy, alkoxy, aryloxy, amino, alkylamino, dialkylamino, arylamino, diarylamino, cabroxy, carboalkoxy, carbonamido (all of which may contain up to 30 carbon atoms, preferably up to 12 carbon atoms), sulfonic acid, sulfonate, aryl-sulfonyl, sulfoalkoxy, sulfonamido, halide, e.g. fluorine, chlorine, bromide, iodine, and

n is an integer between 0 and 4.

Dye-forming developers of the amino naphthol type suitable for use in the invention include those of the general formula:

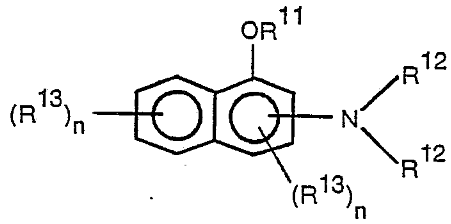

(3)

in which $R^{11}$, $R^{13}$ and n are as defined above in formula (2), the amino group can be either in the 2 or 4 position, and each $R^{12}$ is as defined above in formula (2) or together represent the necessary atoms to form a heterocyclic ring such as 2,5-dialkylpyryl, 2,6-dialkyl-1,4-oxazolyl and 4-oxo-pyridyl.

Dye-forming developers of the alkyl-1-naphthol type include those of the general formula:

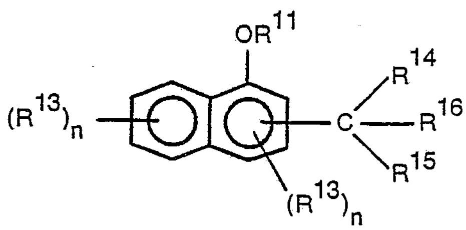

(4)

in which the $CR^{14}R^{15}R^{16}$ group can be in the 2 or 4 position, $R^{11}$, $R^{13}$ and n are as defined above, $R^{14}$ represents alkyl (of up to 20 carbon atoms) or preferably hydrogen,

$R^{15}$ is hydrogen, alkyl (of up to 20 carbon atoms) or preferably an aromatic group, e.g. phenyl, *p*-hydroxyphenyl, *p*-tolyl, *p*-anisyl, xylyl, mesityl, *p*-dialkylaminophenyl, *p*-biphenyl, 1-naphthyl, 2-naphthyl, 9-anthracenyl and phenanthryl,

$R^{16}$ is preferably an aromatic group, said group being capable of activating the methine hydrogen of the naphthol developer when either $R^{14}$ or $R^{15}$ represents hydrogen e.g. aryl, alkylaryl, alkoxyaryl, hydroxyaryl, tropyl or $R^{16}$ together with $R^{15}$ and their linking carbon atom represents the necessary atoms to complete a carbocyclic or heterocyclic ring system which is fused or linked to one or more aromatic rings other than the naphthalene nucleus. Such a $CR^{15}R^{16}$ ring can assume the following general structures: fluorenyl, anthryl, benzanthryl, dibenzosuberyl, xanthyl, thioxanthyl, acridyl, diarylcyclopropenyl, tropyl, dibenzotropyl, arylchromyl, arylthiochromyl, chromyl, thiochromyl, 2,3-diaryl-1,4-imidazolyl, and includes:

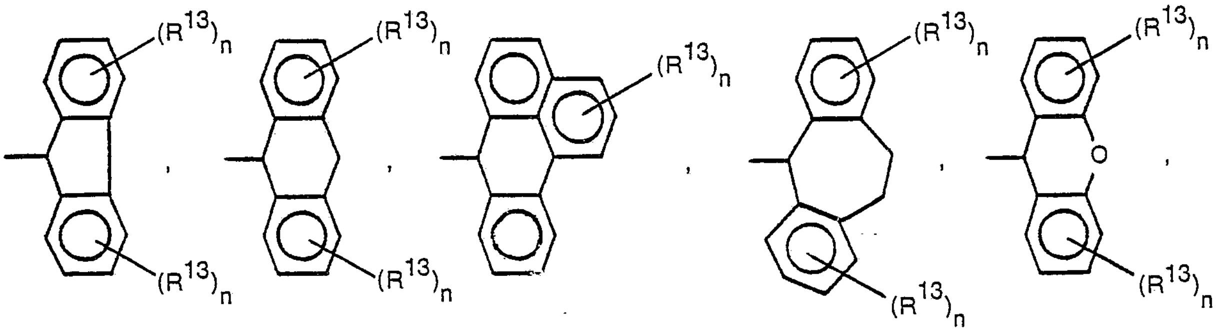

, , , ,

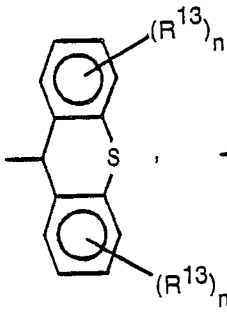 , 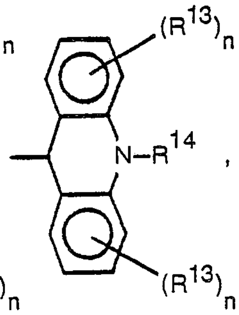 , 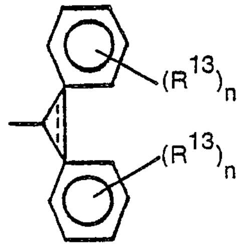 , 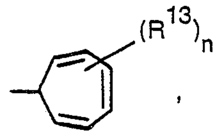 ,

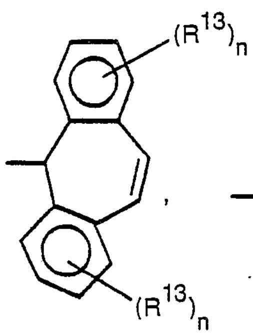 , 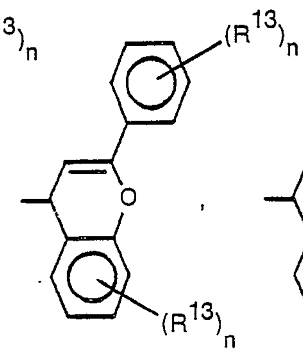 , 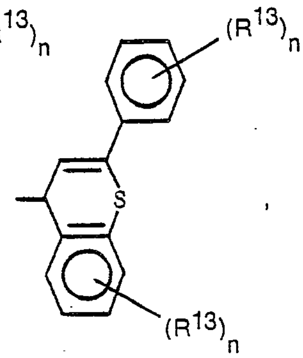 ,

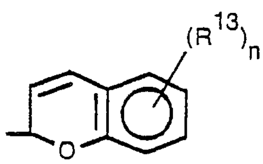 , 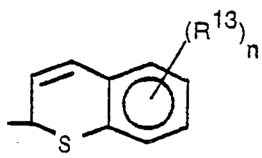 , 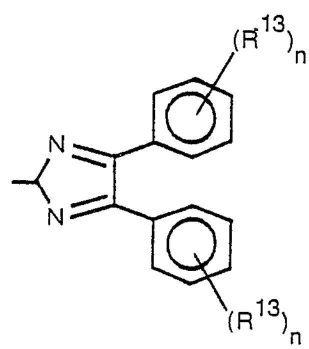

in which $R^{13}$, $R^{14}$ and n are as defined above.

The above naphthol developers must have at least one aromatic group among the $CR^{14}R^{15}R^{16}$ moieties and where there are two hydroxyl groups on either rings of the naphthols they can have two $CR^{14}R^{15}R^{16}$ groups preferably $\alpha$ and $\gamma$ to the hydroxyl groups. The ring substituent $R^{13}$ can be alkali solubilizing group such as hydroxyl but it is not essential that the naphthol developers of the present invention possess two hydroxyl groups.

Polynuclear hydroquinones and their monoethers which are useful in the practice of this invention correspond to the general formulae:

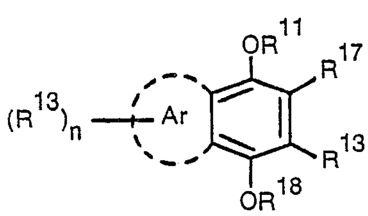 (5)

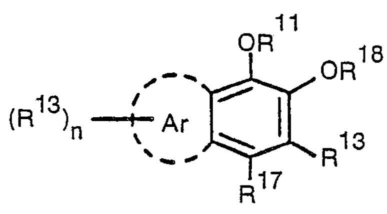 (6)

in which $R^{11}$, $R^{13}$ and n are as defined above,

$R^{17}$ represents hydrogen, alkyl, aryl, alkylaryl, alkoxyaryl, hydroxyaryl, aminoaryl, dialkylaminoaryl, and a combination thereof or forms a furan ring with the $\alpha$-hydroxy group,

$R^{18}$ represents hydrogen, alkyl, arylalkyl, alkoxy-alkyl, aminoalkyl, quaternary ammonium alkyl or alkyl sulfonate (preferably with up to 20 carbon atoms in each, more preferably with 1 to 8 carbon atoms in the alkyl groups and most preferably with 1 to 3 carbon atoms in the alkyl groups and phenyl for aryl).

The polynuclear Ar group can be any fused aromatic or heterocyclic ring including benzo, naphtho and having the following structures:

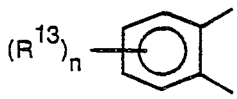
,
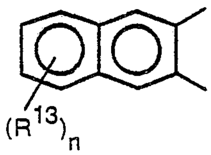
,
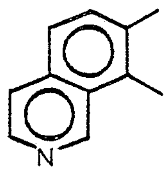
,
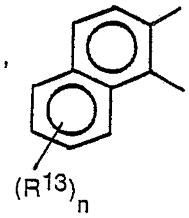
,
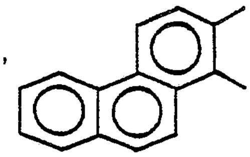
,
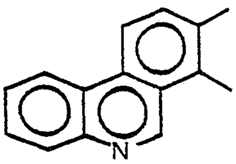
,
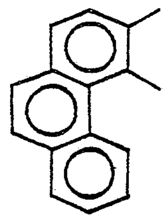
,
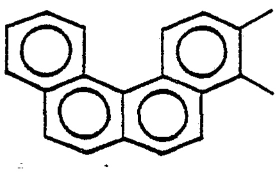
,
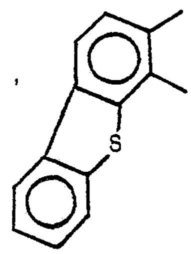
,
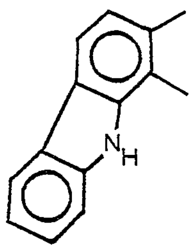
;
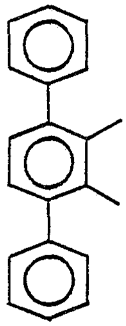
,
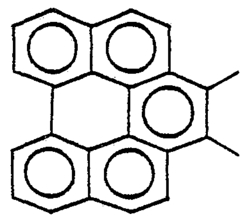

Heterocyclic hydroquinones, naphthohydroquinones and precursors which are useful in the practice of this invention correspond to the following formulae:

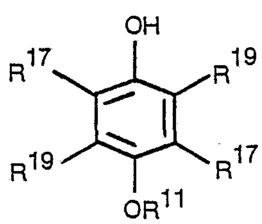
(7)

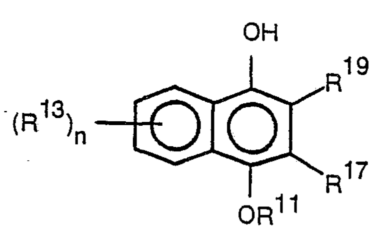
(8)

in which $R^{11}$, $R^{13}$, $R^{17}$ and n are as defined above,

$R^{19}$ is preferably an aryl group (preferably up to 20 carbon atoms, most preferably phenyl) or together with $R^{17}$ represents the necessary atoms to complete a heterocyclic ring selected from amongst the following structures:

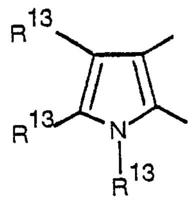 , 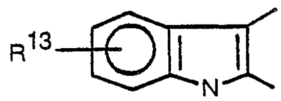 , 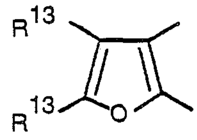 , 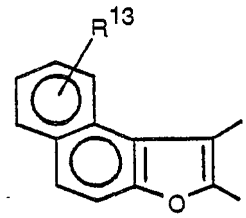 ,

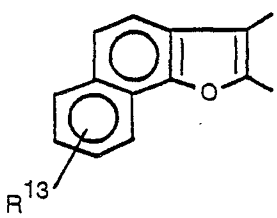 , 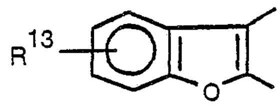 , 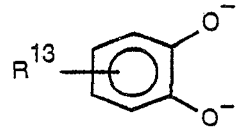 , 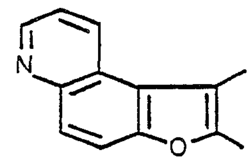 ,

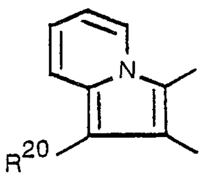 , 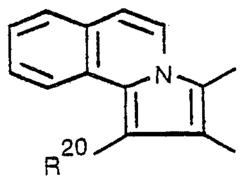 , 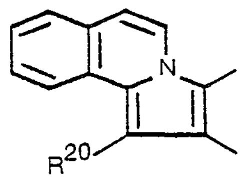 , 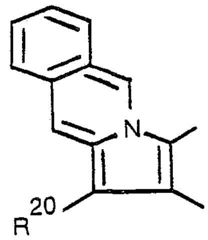 ,

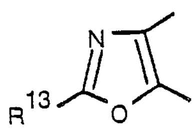 , 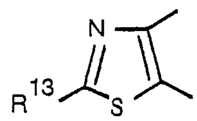 , 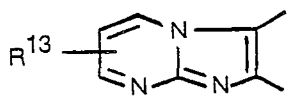 .

$R^{20}$ represents alkanoyl, aroyl, cyano, aryl or the like.

Bisphenols useful as dye-forming developing agents in this invention correspond to the general formulae:

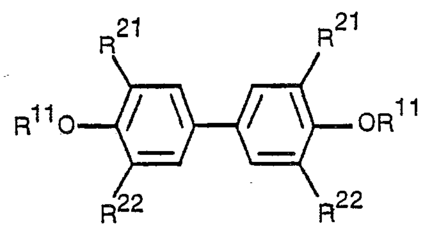

(9)

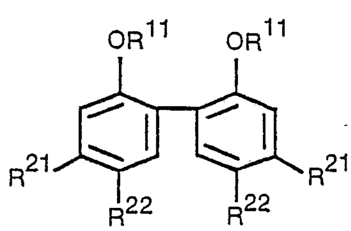

(10)

in which $R^{11}$ is as defined above,

$R^{21}$ is alkyl, alkoxy, aryl dialkylamino,

$R^{22}$ is alkyl, aryl, alkoxy, dialkylamino or together with $R^{21}$ represents the necessary atoms to form an alicyclic, oxymethylene or aromatic ring.

All alkyl and alkoxy groups, including those on the amines, all preferably 1 to 30 carbon atoms, 1 to 20 carbon atoms, more preferably 1 to 12 carbon atoms and most preferably 1 to 3 carbon atoms. Aryl groups are preferably up to 30 carbon atoms, more preferably up to 15 carbon atoms and most preferably phenyl.

Bis-$\alpha$-naphthols useful as dye-forming developing agents of this invention correspond to the following formulae:

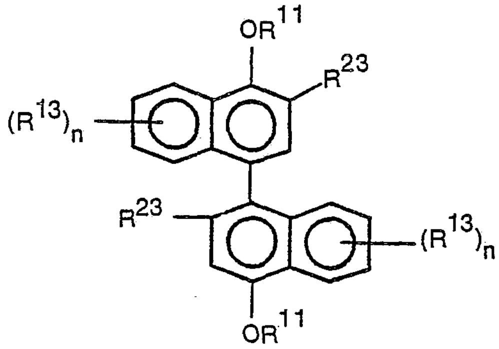

(11)

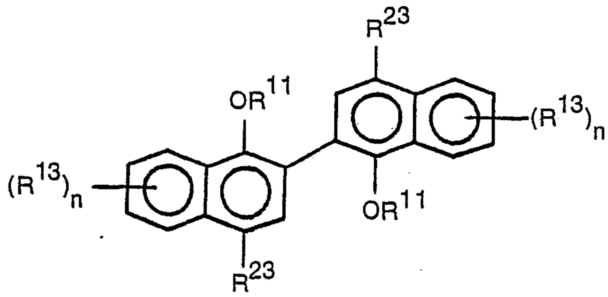

(12)

in which $R^{11}$, $R^{13}$ and n are as defined above.

$R^{23}$ represents hydrogen, alkyl, arylalkyl, alkoxy, aryloxy, alkylaryloxy, aryl, alkylaryl, alkoxyaryl, hydroxyaryl, aminoaryl, alkyl and dialkylaminoaryl, carboalkoxy, carboaryloxy, carbonamido, alkylamino, arylamino, diarylamino, N-heterocyclic.

2-Naphthols useful as developing agents of the invention correspond to the following formula:

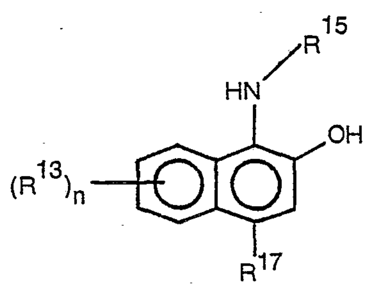

(13)

in which $R^{13}$, $R^{15}$, $R^{17}$ and n are as defined above.

Amino naphthohydroquinone developer precursors (keto-1,3-naphthoxazoline), useful in this invention corresponding to the general formula:

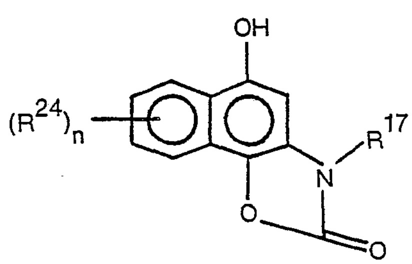

(14)

in which $R^{17}$ and n are as defined above, and

$R^{24}$ represents hydrogen, alkyl, alkoxy, hydroxy, amino, alkylamino, dialkylamino, N-teterocyclic, aryl or forms a fused aromatic or heterocyclic ring.

The 4-alkoxy-1-naphthols employed in this invention can be prepared according to Japanese Patent Specification No. 70/10338 or United States Patent Specification No. 2,572,822 through reduction of 1,4-naphthoquinone in the presence of stannous chloride, phosphoryl chloride and alcohol.

2-Alkoxy-1-naphthols employed in this invention can be prepared by reduction of 1,2-naphthoquinones in a similar fashion as for the 4-alkoxy analogues or according to J. Chem. Soc. (C), 1969, p. 1982, using 1-bromo-2-alkoxynaphthalene or oxidation of 2-alkoxy-naphthalene with lead tetraacetate.

Masked naphthol developers can be prepared by acylation with an acid anhydride or an acid chloride in the presence of an acid acceptor such as triethylamine, pyridine, collidine, N,N-dimethylaniline.

4-Arylmethyl-1-naphthol developers, typically exemplified by 4-benzyl-1-naphthol can be prepared by Friedel Craft alkylation involving $\alpha$-naphthol, benzyl-bromide and a Lewis acid such as zinc chloride according to J. Chem. Soc. *1952,* 4699, J. Chem. Soc. (C), *1966,* 926; *1971,* 2399, J. Orig. Chem. (1967), *32,* 2941.

The naphthofuchsone dyes can be prepared directly via Wittig reaction according to Tetrah. Lett. *1969,* 457. The dyes can then be converted to the leuco form with a reducing agent such as sodium borohydride sodium dithionite or zinc.

Dialkylamino-1-naphthol developers can be prepared from condensation of amino-1-naphthol or its hydrochloride salt with a diketo compound e.g. acetonyl acetone, dehydroacetic acid or chelidonic acid.

Polynuclear hydroquinone developers can be obtained by reduction of their quinone dyes which are prepared by Diels-Alder reaction between an activated vinylaromatic or polynuclear aromatic hydrocarbon with excess benzoquinone according to J. Amer. Chem. Soc. 1949, 71, 3051, J. Chem. Soc. *1957*, 366, 4951 and Montash. Chem. (1968), *99*, 2032.

Heterocyclic hydroquinone and naphthohydroquinone developers are obtained by reduction of their respective heterocyclic quinone dyes which can be prepared from condensation of 2,3-dichloro-2,4-naphthoquinone or chloranil with phenols, naphthols or activated methylene compounds in the presence of a base such as pyridine, quinoline or triethylamine according to J. Orig. Chem. 1972, *37* (9), 1442; 1963, *28*, 520, 1022; 1957, *22*, 342; 1954, *19*, 176, J. Chem. Soc. 1952, 489, 2699, J. Amer. Chem. Soc. 1957, *79*, 1212, 5489.

*p*-Bisphenols can be prepared from oxidative coupling of phenols according to United States Patent Specification No. 4,097,461.

*O*-Bisphenols can be prepared from reduction of the corresponding *o*-diphenoquinone dyes which are obtained by oxidation of phenols with potassium ferricyanide or ferric chloride according to Tetrah. 1978, 1595, J. Chem. Soc. 1962, 4987, 1968, 1434.

Bis-$\alpha$-naphthol developers can be prepared either by reducing the corresponding dinaphthoquinone dyes with sodium borohydride or by oxidative coupling of 2-alkyl or 2-alkoxy-1-naphthol with ferric chloride.

The following Table 1 reports dye-forming developing agents suitable for use in the present invention and which may be prepared in accordance with the above described methods. "Class" refers to the numbered general formulae listed above.

TABLE 1

| Compound No. | Nomenclature (structure) | Class | Color (max nm) |
|---|---|---|---|
| 1 | 4-ethoxy-1-naphthol | 2 | |
| 2 | 4-propoxy-1-naphthol | 2 | |
| 3 | 4-isopropoxy-1-naphthol | 2 | |
| 4 | 4-butoxy-1-naphthol | 2 | blue |
| 5 | 4(2-chloroethoxy)-1-naphthol | 2 | |
| 6 | 4-(2-methoxyethoxy-1-naphthol | 2 | |
| 7 | 4-cyclohexyloxy-1-naphthol | 2 | |
| 8 | 4-benzyloxy-1-naphthol | 2 | blue |
| 9 | 4-furfuryloxy-1-naphthol | 2 | |
| 10 | 2-bromo-4-methoxy-1-naphthol | 2 | |
| 11 | 2-chloro-4-methoxy-1-naphthol | 2 | |
| 12 | 2,4-dimethoxy-1-naphthol | 2 | |
| 13 | 4-methoxy-5-methoyl-1-naphthol | 2 | |
| 14 | 4-methoxy-8-methyl-1-naphthol | 2 | |
| 15 | 4-methoxy-8-hydroxy-1-naphthol | 2 | |
| 16 | 4,8-dimethoxy-1-naphthol | 2 | |
| 17 | 4-methoxy-7-ethoxy-1-naphthol | 2 | |
| 18 | 4-methoxy-7-methyl-1-naphthol | 2 | |
| 19 | 2-methyl-4-methoxy-7-ethoxy-1-naphthol | 2 | |
| 20 | 5-acetoxy-8-methoxy-1-naphthol | 2 | |
| 21 | 1-acetoxy-4-methoxy-5-acetylnaphthalene | 2 | |
| 22 | 4-phenoxy-1-naphthol | 2 | |
| 23 | 2-methoxy-1-naphthol | 2 | purple (500) |
| 24 | 4,5-dimethoxy-1-naphthol | 2 | |
| 25 | 4-methoxy-1-anthracenol | 5 | blue |
| 26 | 4-methoxy-9-phenyl-1-anthracenol | 5 | |
| 27 | 4-octyloxy-1-naphthol | 2 | blue |
| 28 | 4-(2-ethoxy)-ethoxy-1-naphthol | 2 | blue |
| 29 | 4-dodecyloxy-1-naphthol | 2 | blue (643) |
| 30 | 4-(2-methacryloyl-oxy)-ethoxy-1-naphthol | 2 | |

TABLE 1 (continued)

| Compound No. | Nomenclature (structure) | Class | Color (max nm) |
|---|---|---|---|
| 31 | 2-benzyloxy-1-naphthol | 2 | purple (545) |
| 32 | 2,5-diphenyl-hydroquinone | 7 | |
| 33 | 2,5-dibenzyl-hydroquinone | 7 | yellow |
| 34 | 2,5-di(2,4-dimethylphenyl)-hydroquinone | 7 | |
| 35 | 2,5-di(2,4,6-trimethyl-phenyl) hydroquinone | 7 | |
| 36 | 2-(4-methylphenyl)-hydroquinone | 7 | |
| 37 | 2-(4-methoxyphenyl)-hydroquinone | 7 | yellow (425) |
| 38 | 2-(2,4-dimethoxyphenyl)-hydroquinone | 7 | yellow (440) |
| 39 | 2-(4-methoxy-phenyl)-5-benzene sulfonyl hydroquinone | 7 | yellow |
| 40 | 2-(2,4-dimethoxyphenyl)-5-benzene-sulfonyl-hydroquinone | 7 | yellow |
| 41 | 2-diphenylamino-5-phenyl-hydroquinone | 7 | purple |
| 42 | 2-(N-ethyl-N-phenyl-amino)-5-phenyl-hydroquinone | 7 | purple |
| 43 | 4-methyl-1-naphthol | 4 | orange-red (504) |
| 44 | 4-cyclohexyl-1-naphthol | 4 | |
| 45 | 4-benzyl-1-naphthol | 4 | |
| 46 | 4-isopropyl-1-naphthol | 4 | |
| 47 | 4-diphenylmethyl-1-naphthol | 4 | yellow (398) |
| 48 | 4-phenyl-1-naphthol | 4 | blue |
| 49 | 2-benzyl-1-naphthol | 4 | red |
| 50 | 2-benzyl-6-methoxy-1-naphthol | 4 | |
| 51 | 2-cyclohexyl-1-naphthol | 4 | red |
| 52 | 2-(4-methylbenzyl)-6-methoxy-1-naphthol | 4 | |
| 53 | 2-(4-methylbenzyl-1-naphthol | 4 | |
| 54 | 2-t-butyl-1-naphthol | 4 | orange (490) |
| 55 | 2-methyl-1-naphthol | 4 | orange (492) |
| 56 | 4-methyl-1-anthracenol | 4 | |
| 57 | 4-methyl-9-phenyl-1-anthracenol | 4 | |
| 58 | 2-(9-fluorenyl)-1-naphthol | 4 | purple (516) |

TABLE 1 (continued)

| Compound No. | Nomenclature (structure) | Class | Color (max nm) |
|---|---|---|---|
| 59 | 2-diphenylmethyl-1-naphthol | 4 | purple |
| 60 | 2-(1-phenylethyl)-1-naphthol | 4 | purple (504) |
| 61 | 2-(4-methoxybenzyl)-1-naphthol | 4 | purple |
| 62 | 3,5,3′,5′-tetramethyl-4,4′-dihydroxy-[1,1′-biphenyl] | 9 | yellow (420) |
| 63 | 3,5,3′,5′-tetraisopropyl-4,4′-dihydroxy-[1,1′-biphenyl] | 9 | yellow (420) |
| 64 | 3,5,3′,5′-tetra-s-butyl-4,4′-dihydroxy-[1,1′-biphenyl] | 9 | yellow (423) |
| 65 | 3,5,3′,5′-tetra-t-butyl-4,4′-dihydroxy-[1,1′-diphenyl] | 9 | yellow (421) |
| 66 | 3,5,3′,5′-tetramethoxy-4,4′-dihydroxy-[1,1′-biphenyl] | 9 | yellow (460) |
| 67 | 3,5,3′,5′-tetraphenyl-4,4′-dihydroxy-[1,1′-biphenyl] | 9 | |
| 68 | 3,3′-dimethoxy-5,5′-di- -styryl-4,4′-dihydroxy-[1,1′-biphenyl] | 9 | purple |
| 69 | 4,5,4′,5′-tetramethyl-2,2′-dihydroxy-[1,1′-biphenyl] | 10 | |
| 70 | 3,5,3′,5′-tetramethyl-2,2′-dihydroxy-[1,1′-diphenyl] | 10 | |
| 71 | 4,5,4′,5′-tetramethoxy-2,2′-dihydroxy-[1,1′-diphenyl] | 10 | purple |
| 72 | 3,5,3′,5′-tetra-t-butyl-2,2′-dihydroxy-[1,1′-diphenyl] | 10 | |
| 73 | 4,5,4′,5′-bis-methylene-dioxy-2,2′-dihydroxy-[1,1′-biphenyl] | 10 | purple (552) |
| 74 | 3,3′-di-t-butyl-5,5′-dimethoxy-2,2′-dihydroxy-[1,1′-biphenyl] | 10 | |
| 75 | 4,4′-di-t-butyl-5,5′-dimethoxy-2′-dihydroxy-[1,1′-biphenyl] | 10 | |
| 76 | 3,4,3′,4′-tetramethyl-5,5′-dimethoxy-2,2′-dihydroxy-[1,1′-biphenyl] | 10 | |
| 77 | 1,1′-dihydroxy-2,2′-binaphthyl | 12 | |
| 78 | 1,1′-dihydroxy-4,4′-dimethyl-2,2′-binaphthyl | 12 | purple (554) |
| 79 | 1,1′-dihydroxy-4,4′-dimethoxy-2,2′-binaphthyl | 12 | blue (622) |

TABLE 1 (continued)

| Compound No. | Nomenclature (structure) | Class | Color (max nm) |
|---|---|---|---|
| 80 | 1,1′-dihydroxy-4,4′-di(triphenyl-methyl) 2,2′ binaphthyl | 12 | blue |
| 81 | 1,1′-dihydroxy-4,4′-di-dodecyloxy-2,2′-binaphthyl | 12 | blue (643) |
| 82 | 4,4′-dihydroxy-1,1′-binaphthyl | 11 | purple |
| 83 | 3,3′-dimethoxy-4,4′-dihydroxy-1,1′-binaphthyl | 11 | purple (545) |
| 84 | 3,3′-di-t-butyl-4,4′-dihydroxy-1,1′-biphenyl | 11 | orange (490) |
| 85 | 3,3′-di(9-fluorenyl)-4,4′-dihydroxy-1,1′-biphenyl | 11 | red (516) |
| 86 | 3,3′-di-diphenylmethyl-4,4′-dihydroxy-1,1′-diphenyl | 11 | red |
| 87 | 4,4′-diphydroxy-1,1′-diphenyl | 9 | |
| 88 | 3,3′-dimethoxy-4,4′-dihydroxy-1,1′-biphenyl | 9 | |
| 89 | 4-amino-1-naphthol | 3 | |
| 90 | 1-amino-2-naphthol | 13 | |
| 91 | 4-(2,5-dimethylpyrrolyl)-1-naphthol | 3 | |
| 92 | 4-benzylideneanil-1-naphthol | 3 | |
| 93 | 1,4-bis[4-hydroxy-3,5-di-t-butyl-phenyl]-2,3-dicyano-1,3-butadiene | | red (514) |
| 94 | 3,5,3′,5′-tetra-t-butyl-4,4′-dihydroxy-stilbene | | yellow (458) |
| 95 | 2,5-di(3-methoxy-4-hydroxyphenyl) furan | | blue |
| 96 | bis(3,5-di-t-butyl-4-hydroxy-benzylidene) azine | | |
| 97 | bis(3-methoxy-4-hydroxybenzylidene)azine | | |
| 98 | 2,6-dimethyl-4-diphenylmethylphenol | | yellow |
| 99 | 2,6-di-t-butyl-4-diphenylmethylphenol | | yellow |
| 100 | 2,6-di-methoxy-4-diphenylmethylphenol | | yellow |
| 101 | 2-benzyl-1,4-dihydroxynaphthalene | 8 | yellow |
| 102 | 2-methoxy-1,4-dihydroxynaphthalene | 8 | yellow |
| 103 | 2-(2,4-dihydroxyphenol)-1,4-dihydroxynaphthalene | 8 | |
| 104 | 2-(2,4-dimethoxyphenyl)-1,4-dihydroxynaphthalene | 8 | yellow |

TABLE 1 (continued)

| Compound No. | Nomenclature (structure) | Class | Color (max nm) |
|---|---|---|---|
| 105 | 3-hydroxyphenothiazine | | purple (535) |
| 106 | 1-phenylamino-2-naphthol | 13 | |
| 107 | 4-phenylamino-1-naphthol | 3 | |
| 108 | 3-phenylamino-1-naphthol | 3 | |
| 109 | 2-methoxy-1-anthracenol | 6 | purple |
| 110 | 2-phenyl-1-naphthol | 4 | purple |
| 111 | 2-phenyl-1,4-dihydroxynaphthalene | 8 | |
| 112 | 2,5-diphenoxyhydroquinone | 7 | |
| 113 | 3,5,3′,5′-tetrachloro-4,4′-dihydroxy-1,1′-biphenyl | 9 | |
| 114 | 3,5,3′,5′-tetrachloro-4,4′-dihydroxy-1,1′-biphenyl | 9 | |
| 115 | 3-hydroxyphenoxazine | | |
| 116 | 1-trichloroacetoxy-4-methoxynaphthalene | 2 | blue (622) |
| 117 | N-[1-(4-hydroxy)-naphthyl]-2,5-dimethylpyrrolhydrochloride | 3 | |
| 118 | N-[1-(4-hydroxy)naphthyl]-pyridone-hydrochloride | 3 | |
| 119 | N-[1-(4-hydroxy)naphthyl<-2,6-di-methyl-dihydromorpholine hydrochloride | 3 | |
| 120 | 1-(4-methoxyphenethyl)-1-naphthol | 4 | red (509) |
| 121 | 2-biphenethyl-1-naphthol | 4 | red (503) |
| 122 | 2-[1-(2-naphthyl)ethyl]-1-naphthol | 4 | red (506) |
| 123 | 2-benzyl-1,7-dihydroxynaphthalene | 4 | |
| 124 | 2-diphenylmethyl-1,7-dihydroxynaphthalene | 4 | |
| 125 | 2-(9-dibenzosuberyl)-1-naphthol | 4 | red |
| 126 | 2-(9-dibenzotropyl)-1-naphthol | 4 | |
| 127 | 1,4-dihydroxychrysene | 5 | yellow |
| 128 | 1,4-dihydroxybenzophenanthrene | 5 | |
| 129 | 1,4-dihydroxy-6-methoxy-naphthalene | 5 | |
| 130 | 1,4-dihydroxy-10-methoxynaphthalene | 5 | |
| 131 | dinaphtho-[2,3;2′,3′]-furan-8,13-diol | 8 | yellow |

TABLE 1 (continued)

| Compound No. | Nomenclature (structure) | Class | Color (max nm) |
|---|---|---|---|
| 132 | 1,1'-dihydroxy-2,2'-dibenzyloxy-4,4'-dinaphthalene | 11 | purple (545) |
| 133 | 2-(4-dimethylaminophenyl)-1,4-dihydroxy naphthalene | 8 | |
| 134 | 1,1'-dihydroxy-2,2'-dibenzyl-4,4'-dinaphthyl | 11 | red |
| 135 | 2-(2,4,5-trimethoxyphenyl)-1,4-dihydroxynaphthalene | 8 | yellow |
| 136 | 2-(2-hydroxy-4-methoxyphenyl)-1,4-dihydroxynaphthalene | 8 | yellow |
| 137 | 2-[1-(4-hydroxy)naphthyl<-1,4-dihydroxynaphthalene | 8 | yellow |
| 138 | 4-[1-(4-hydroxy)-naphthyl]-1,2-dihydroxynaphthalene | 6 | yellow |
| 139 | 4-(2,4-dimethoxyphenyl)-1,2-dihydroxynaphthalene | 6 | yellow |
| 140 | 4-(2,4,5-trimethoxyphenyl)-1,2-dihydroxynaphthalene | 6 | yellow |
| 141 | 4-(p-dimethylaminophenyl)-1,2-dihydroxynaphthalene | 6 | |
| 142 | 1,1'-dihydroxy-2,2'-dimethyl-4,4'-dinaphthyl | 11 | |
| 143 | 1,1-dihydroxy-4,4'-di[n-(2,5-dimethyl)-pyrrolyl]-2,2'-dinaphthyl | | |
| 144 | 1,1'-dihydroxy-4,4'-di-diethylamino-2,2'-dinaphthyl | | |
| 145 | 1,1'-dihydroxy-4,4'-di[n-2,6-dimethyl)-dihydromorpholinyl]-2,2'-dinaphthyl | | |
| 146 | 2-(9-zanthyl)-1-naphthol | 4 | |
| 147 | 1-hydroxy-4-methoxychrysene | 5 | |
| 148 | 4-diethylamino-1-naphthol | 3 | |
| 149 | 2-methyl-1-naphthol | 4 | |
| 150 | 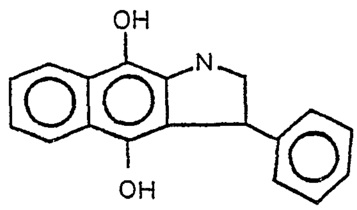 | 8 | |

TABLE 1 (continued)

| Compound No. | Nomenclature (structure) | Class | Color (max nm) |
|---|---|---|---|
| 151 | 2,5-bis-dimethylaminophenyl-hydroquinone | 7 | |
| 152 | 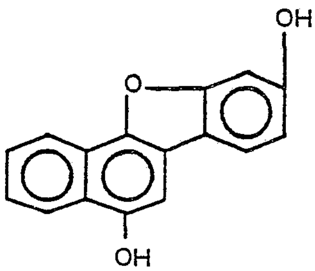 | 2 | |
| 153 | 4-methoxy-1-naphthol | 2 | |

The invention will now be illustrated by the following Examples.

Example 1

A developer solution in accordance with the invention was prepared by mixing the following components in the indicated order:

*Solution A*

| | |
|---|---|
| triethylene glycol | 100 ml |
| diethanolamine | 100 ml |
| 4-methoxy-1-naphthol (MHN) | 7 g |

*Solution B*

| | |
|---|---|
| distilled water | 500 ml |
| sodium carbonate (anhydrous) | 50 g |
| hydroxylamine hydrochloride | 10 g |
| metol (methyl-p-aminophenol sulfate) | 1 g |
| sodium sulfite (anhydrous) | 10 g |
| potassium bromide | 5 g |
| sodium hydroxide (1 M solution) | 150 ml |

The addition of MHN to a warm (30°C) triethyleneglycol/diethanolamine mixture, followed by stirring at room temperature facilitated the solubility of the former in the organic solvent system.

If the pH of Solution B was lower then 11.3 adjust using sodium hydroxide 1M solution to a pH of 11.3 to 11.5.

Solution A was added to Solution B under stirring and the volume was made up to 1000 ml with distilled water. The final pH was adjusted to 11 ± 1.

Optimum development time was dependent upon the characteristics of the emulsion tested and the acceptable contrast, density, speed and fog.

As a general guide:
development time 2 to 2-1/2 minutes at 28°C
45 seconds at 37°C
1-1/2 minutes at 32°C.

The fixer used was RP—Xomat fixer for a period of 30 to 60 seconds at 20°C.

An iodobromide photographic emulsion suitable for microfilm photographic use was coated at coating weights of 2.2 g/$m^2$ and 1.2 g/$m^2$. Samples of both coatings were exposed behind a 0 to 4 log E neutral continuous sensitometric wedge. The samples were then processed in the above developer.

Example 2

A developer solution in accordance with the invention was prepared in a similar manner to that in Example 1 by mixing the following components in the indicated order:

*Solution A*

| | |
|---|---|
| triethyleneglycol | 100 ml |
| diethanolamine | 100 ml |
| 4-methoxy-1-naphthol | 10 g |

*Solution B*

| | |
|---|---|
| distilled water | 400 ml |
| sodium carbonate (anhydrous) | 50 g |
| hydroxylamine hydrochloride | 10 g |
| metol (methyl-p-aminophenol sulfate) | 1 g |
| sodium sulfite (anhydrous) | 10 g |
| potassium bromide | 5 g |
| benzotriazole | 0.3 g |
| sodium hydroxide (1 M solution) | 100 ml |

The pH of Solution B was adjusted to pH 11 and the resulting pH upon mixing solutions A and B was 11.2 $\pm$ 0.1.

The above is a stabilized developer formula containing 4-methoxy-1-naphthol as a silver halide reducing agent and a blue dye former. The image produced by the above developer is the sum total of a silver metal and a blue-dye image formed at exposed area of a silver halide photographic system.

Example 3

This Example illustrates the image amplification obtaining using the developer solution of Example 2 and a range of silver halide photographic films ranging from coarse to fine, iodo- or chlorobromide emulsions prepared in conventional manner, to provide systems suitable for microfilm, graphic arts line film, surveillance film and X-ray film. The emulsions were coated at 40 to 50% lower silver coating weights than standard, and were processed in a standard black and white developer appropriate to every system and the dye forming developer. Comparison of the maximum densities resulting from the two developers and reported in the following Table 2 reflects the amplification factor.

TABLE 2

| Film | Silver coating weight (g/m²) | Dmax with Standard Developer | Dmax with developer of Example 2 |
|---|---|---|---|
| Coarse I/Br (X-ray) Blue sensitive | 3.6 | 1.8 | 3.2 |
| Fine I/Br (Microfilm) Pan sensitive | 1.2 | 1.4 | 2.6 |
| Broad I/Br (Surveillance) Pan sensitive | 2.5 | 1.2 | 2.0 |
| Medium Cl/Br (Line film) Green sensitive | 2.4 | 3.0 | 5.0 |

From the above results, the dye forming developer described in Example 2 functions as a photographic silver halide developer independent of the nature of the photographic emulsion to produce a silver image reinforced by a blue dye image.

Example 4

The stability of developer solutions containing 4-methoxy-1-naphthol as a dye-forming developing agent and various combinations of:

| | |
|---|---|
| triethylene glycol (TRI) | component (1) |
| methanol (MeOH) | component (1) |
| diethanolamine (DEA) | component (2) |
| hydroxylamine hydrochloride (HAC) | component (3) |

was investigated. The above components were used in the formulation of Example 2 in the amounts specified hereinafter.

The developer solutions were stored at room temperature in glass beakers covered with watch-glass covers to minimise evaporation and keep out dirt, and at daily intervals, an X-ray iodobromide emulsion coated on the double sides of a polyester base (3.6 g/m² Ag) and image wise exposed was used to test the variation in developer stability through monitoring the formation of the dye on the developer surface and the variation in the Dmax with time. The development time was 90 seconds at 32°C. The developed film was then fixed, washed and dried.

The following Table 3 reports the presence or absence of the components and the corresponding Dmax.

TABLE 3

| Formulation | 1 | 2 | 3 | 4 | 5 | 6 |
|---|---|---|---|---|---|---|
| MeOH ml/l | 0 | 100 | | 0 | 0 | 100 |
| TRI ml/l | 100 | 0 | 100 | 100 | 0 | 0 |
| DEA ml/l | 100 | 0 | 0 | 100 | 100 | 100 |
| HAC g/l | 10 | 0 | 10 | 0 | 10 | 10 |

| | Dmax using Formulation** | | | | | |
|---|---|---|---|---|---|---|
| Days | 1 | 2* | 3 | 4 | 5 | 6 |
| 0 | 3.40 | 3.71 | 3.50 | 3.40 | 3.50 | 4.00 |
| 1 | 3.40 | 3.75 | 3.40 | 3.40 | 3.50 | 3.80 |
| 2 | 3.20 | 3.47 | 3.40 | 3.20 | 3.10 | 3.80 |
| 5 | 3.30 | 3.76 | 3.10 | 2.20 | 2.20 | 3.80 |
| 7 | 3.30 | 3.44 | 3.10 | 2.40 | 2.00 | 3.70 |
| 13 | 2.20 | 2.25 | 1.50 | 1.60 | 1.40 | 2.60 |
| 15 | 1.80 | 1.68 | 0.60 | 1.00 | 0.90 | 2.00 |
| 19 | 1.20 | 1.01 | 0.30 | 0.40 | 0.50 | 1.10 |
| Dye precipitation | None | | A lot within a few minutes | None | Slight | None |

* The developer produces large amounts of the Russig's Blue dye (the oxidation product of developing agent 4-methoxy-1-naphthol) just on preparation and while standing, making the developer unstable and difficult to use for any reproducible results.
** Dmax measured through a red filter.

Example 5

The stability of developer solutions based on the formulation of Example 2 was examined replacing the hydroxylamine hydrochloride (HAC) content with the following derivatives of HAC:

A) 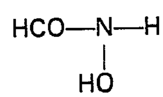

B) 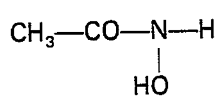

C) 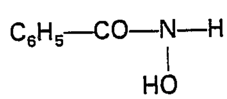

D) 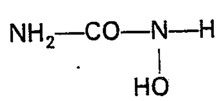

E) $CH_3$—NH—CO—N—H
                         |
                        HO

The developer solutions were tested in an analogous manner to Example 4 and the results are reported in the following Table 4.

TABLE 4

Dmax* using Component

| Days | HAC | A | B | C | D | E |
|---|---|---|---|---|---|---|
| 0 | 3.40 | 3.50 | 3.40 | 3.60 | 3.30 | 3.00 |
| 1 | 3.40 | 3.50 | 3.40 | 3.60 | 3.40 | 2.40 |
| 2 | 3.40 | 3.50 | 3.50 | 3.40 | 3.20 | 2.30 |
| 3 | 3.40 | 3.30 | 3.30 | 2.80 | 3.00 | 2.30 |
| 4 | 3.50 | 2.60 | 2.30 | 2.80 | 2.60 | 2.20 |
| 10 | 2.00 | 1.40 | 1.60 | 1.70 | 1.20 | 0.80 |
| 11 | 1.80 | 1.40 | 1.20 | 1.30 | 1.00 | 0.60 |
| 13 | 1.50 | 1.00 | 0.80 | 1.00 | 0.70 | 0.20 |
| Dye precip-itation | None | None | None | None | Slight | Slight |

* Dmax measured with white light — the sum density of the silver and blue dye image.

The results show the versatility of the formulation, where hydroxylamine can be replaced by a certain class of its derivatives to give working developer formulations as shown by the variation of the Dmax with time.

## Example 6

The stability of developer solutions based on the formulation of Example 2 was examined replacing the trigol with the following polar solvents:

A) Ethylene glycol
B) $(HOCH_2CH_2)_2O$
C) Glycerol
D) $(CH_3OCH_2CH_2)_2O$
E) Diethylene glycol ethyl ether
F) Glycol ethyl ether

The developer solutions were tested in an analogous manner to Example 4 and the results are reported in the following Table 5.

TABLE 5

| Days | Dmax* using solvent component A | B | C | D | E | F |
|---|---|---|---|---|---|---|
| 0 | 3.60 | 3.40 | 3.30 | 3.12 | 3.05 | 3.22 |
| 1 | 3.70 | 3.40 | 3.60 | 3.14 | 3.12 | 3.17 |
| 2 | 3.50 | 3.40 | — | 2.85 | 3.29 | 2.71 |
| 5 | 3.20 | 3.40 | 3.40 | 3.25 | 2.50 | 2.37 |
| 7 | 3.50 | 3.20 | 3.40 | 2.38 | 1.91 | 1.42 |
| 13 | 3.00 | 2.00 | 2.30 | 1.47 | 1.06 | 0.95 |
| 15 | 2.40 | 1.80 | 2.80 | — | — | — |
| A | 1.50 | 1.60 | 1.10 | — | — | — |
| Dye precipitation | None | None | None | None | None | Dye ppt on 6th day |

* Dmax measured with white light — the sum density of silver and blue dye image.

The results show the versatility of the developer formulation where the organic water-miscible polar solvent (triethylene glycol) can be replaced by other solvents of similar properties to give a working developer system as shown by the variation of the Dmax with time. Solvents used are preferably of low volatility to reduce their evaporation when the developer is used at high temperatures.

Example 7

A developer solution incorporation *bis*-p,p(2,6-dimethyl) phenol of the formula:

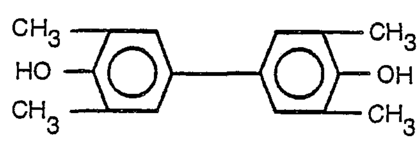

was prepared by mixing the following components in the order indicated:

*Solution A*

| | |
|---|---|
| Triethylene glycol | 100 ml |
| sodium hydroxide (1 M solution) | 100 ml |
| bis-p,p(2,6-dimethyl)phenol | 10 g |

*Solution B*

| | |
|---|---|
| distilled water | 300 ml |
| sodium carbonate (anhydrous) | 50 g |
| N-formylhydroxamic acid | 10 g |
| β-alanine | 20 g |
| potassium bromide | 5 g |
| sodium sulfite | 10 g |
| benzotriazole | 0.3 g |

The pH of solution B was adjusted to pH = 12.2 with solid NaOH and the solutions mixed. The resulting composition was made up to 1 litre with distilled water and the pH adjusted to 12.2.

The developer solution was tested on the X-ray film used in Example 3 and a comparative test made using the same formulation except that $\beta$-alanine and N-formylhydroxamic acid were excluded. The results are reported in the following Table 6.

TABLE 6

Dmax ***

| Days | Formulation of invention | | Comparison |
|---|---|---|---|
| | * | ** | * |
| 0 | 4.38 | 3.86 | 3.06 |
| 1 | 4.42 | 3.94 | 3.54 |
| 4 | 3.49 | 3.01 | 3.03 |
| 5 | 3.38 | 2.45 | 3.03 |
| 6 | 3.02 | 2.52 | 2.80 |
| 8 | 2.63 | 2.24 | 2.34 |
| 13 | 2.13 | 1.92 | 1.06 |
| Dye precipitation | None | None | Yes |

* Dmax measured through a blue filter
** Dmax measured with white light
*** Dmax is the sum total of the silver and yellow dye image densitites.

The results show that the use of bis-p,p(2,6-dimethyl)phenol, which is an example of another class of dye-forming developing agents capable of forming a yellow dye, together with N-formylhydroxamic acid and $\beta$-alanine give a working stable system as monitored by the variation of the Dmax and dye precipitation with time.

Example 8

The stability of developer solutions based on the formulation of Example 2 was examined replacing the diethanolamine with the following amines:

A) triethanol amine
B) ethanolamine
C) 2-diethylaminoethanol
D) $\beta$-alanine

The developer solutions were tested in an analogous manner to Example 3 and the results are reported in the following Table 7.

TABLE 7

| Days | Dmax* using amine A | B | C | D |
|---|---|---|---|---|
| 0 | 3.40 | 3.30 | 3.10 | 3.80 |
| 1 | 3.20 | 3.40 | 3.20 | 3.60 |
| 5 | 3.10 | — | — | — |
| 7 | — | 3.00 | 3.20 | 3.40 |
| 8 | — | 2.40 | 2.40 | 3.00 |
| 15 | 1.60 | 0.80 | 1.80 | 2.20 |
| 19 | 1.30 | — | 0.80 | 1.00 |
| Dye precipitation | None | None | None | None |

* Dmax measured with white light — the sum density of the silver and blue dye image.

The results show the versatility of the formulation where diethanol amine can be replaced with other amines of similar nature to give a working system as shown by the variation of the Dmax with time.

Example 9

The stability of developer solutions based on the formulation of Example 2 was examined replacing the trigol and/or diethanolamine with another polar organic solvent or amine. The solvents and amines used were:

A) ethylene glycol
B) diethanol amine
C) triethylene glycol
D) glycine
E) 1-amino-2-propanol
F) 2-amino-2-methyl-1,3-propandiol
G) $\beta$-alanine.

The developer solutions were tested in an analagous manner to Example 3 and the results are reported in the following Table 8.

TABLE 8

| | Dmax* using solvent/amine | | | | |
|---|---|---|---|---|---|
| | A | C | C | C | A |
| Days | B | D | E | F | G |
| 0 | 3.60 | 3.60 | 3.60 | 3.70 | 3.20 |
| 1 | 3.60 | 3.50 | 3.50 | 3.50 | 3.00 |
| 2 | 3.40 | 3.50 | 3.50 | 3.40 | 3.20 |
| 5 | 3.10 | 3.40 | 3.00 | 3.40 | 3.20 |
| 7 | 3.10 | 2.70 | 2.40 | 3.20 | 2.90 |
| 13 | 1.50 | 2.60 | 1.80 | 2.80 | 2.20 |
| 15 | 0.60 | 1.40 | 1.00 | 1.40 | 0.80 |
| Dye-precipitation | None | None | None | None | None |

* Dmax measured with white light — the sum density of the silver and blue dye images.

The results show the versatility of the formulation where either/or both of diethanol amine and trigol can be replaced with other alkanol amines or polar water-miscible organic solvents to give a working system as monitored by the variation of the Dmax with time.

Example 10

The developer solution of Example 2 was used to develop an image-wise exposed X-ray photographic film as used in Example 3 and the film was bleached using a ferricyanide color bleach solution (similar to that used in conventional color processing) to give rise to a blue-dye, silver free, image. The densities resulting from development with and without bleaching were measured and reported in the following Table 9.

TABLE 9

| Light | Dmax of blue dye and silver image | Dmax of blue dye image |
|---|---|---|
| White | 3.00 | 2.88 |
| Red | 3.47 | 3.28 |

The above results display the possibility of using dye-forming developers of this invention to form silver free dye images comparable to conventional dye images formed from conventional color coupler processes.

Example 11

An imagewise exposed X-ray film was developed using the developer solution of Example 7 and the film was bleached using a ferricyanide color bleach solution (similar to that used in conventional color processing) to give rise to a deep yellow silver-free image. The densities resulting from the development with or without bleaching were measured and are reported in the following Table 10.

TABLE 10

| Light | Dmax<br>Silver and yellow dye image | <br>Yellow dye image |
|---|---|---|
| White | 3.70 | 2.18 |
| Blue | 4.10 | 2.86 |

This Example displays another possibility of using dye-forming developers of this invention to form silver-free dye images comparable to conventional dye images formed from conventional color coupler processes.

Example 12

A developer solution including, as a dye-forming developing agent (DFD), a compound of the formula:

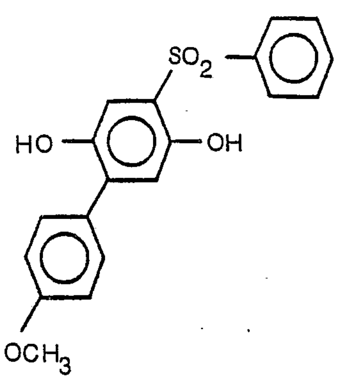

was formed from the following components:

*Solution A*

| | |
|---|---|
| triethylene glycol | 15 ml |
| DFD | 1 g |

*Solution B*

| | |
|---|---|
| distilled water | 30 g |
| sodium carbonate | 5 g |

*Solution C*

| | |
|---|---|
| hydroxylamine hydrochloride | 1 g |
| metol | 0.1 g |
| potassium bromide | 0.5 g |

Solution B was added to Solution A under stirring and thereafter solution C added. The solution was made up to 100 ml with water and the pH adjusted to 10.5 using sodium hydroxide.

The developer solution was used to process an imagewise exposed X-ray film as in Example 3, development time 90 seconds at 32°C. As a comparison the same film was processed in a standard black and white developer to form a dye-free silver image. The densities obtained by processing with the above solutions were measured and are reported in the following Table 11.

TABLE 11

| | Dmax | |
|---|---|---|
| Light | Silver image | Silver and yellow dye image |
| White | 1.78 | 1.69 |
| Blue | 1.80 | 2.61 |

Example 13

The procedures of Example 12 was repeated but the silver image of the X-ray photographic coating which was processed in the yellow dye-forming developer, was bleached using a ferricyanide color bleach solution (similar to that used in conventional color processing) to give rise to a yellow dye, silver-free, image. The densities resulting from development with and without bleaching were measured and are reported in the following Table 12.

TABLE 12

| | Dmax | |
|---|---|---|
| Light | Silver and yellow dye image | Yellow dye image |
| White | 1.69 | 1.25 |
| Blue | 2.61 | 2.15 |

This Example displays the possibility of using a dye-forming developer of another class of compounds to form a silver-free dye image comparable to dye images formed from conventional color coupler processes.

Example 14

A developer solution including, as a dye-forming developing agent (DFD), a compound of the formula:

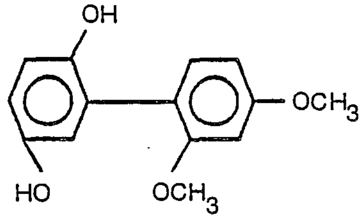

was prepared from the following components:

*Solution A*

| | |
|---|---|
| triethylene glycol | 100 ml |
| diethanolamine | 50 ml |
| DFD | 10 g |

*Solution B*

| | |
|---|---|
| distilled water | 500 ml |
| hydroxylamine-hydrochloride | 10 g |
| metol | 1 g |
| potassium bromide | 5 g |
| sodium hydroxide | 5 g |

Solution A was added to solution B and the resulting solution made up to 1000 ml with water and the pH adjusted to 12.2.

The developer solution was used to process an imagewise exposed X-ray film as in Example 3, development time 15 seconds at 43°C. During the processing the film was bleached using a ferricyanide silver color bleach to give a cream-colored dye, silver-free, image. The densities obtained by processing with the above solution, with or without bleaching were measured and are reported in the following Table 13.

TABLE 13

| | Dmax | |
|---|---|---|
| Light | Silver and cream dye image | Cream dye image |
| White | 1.26 | 1.11 |
| Blue | 1.46 | 1.29 |

Example 15

A developer solution was prepared identical to that used in Example 2 with the exception that tap water was used in place of distilled water and the formulation additionally contained 0.2 g of the sodium salt of ethylenediaminetetraacetic acid as a sequestering agent (formulation 15). An imagewise exposed silver halide photographic film was processed in the above formulation and in the formulation of Example 2 to yield a blue-dye reinforced silver image. The densities were measured and are reported in the following Table 14.

TABLE 14

| Formulation | Dmax using white light |
|---|---|
| Example 2 | 3.80 |
| Formulation 15 | 3.79 |

This Example shows that a dye-forming developer formulation containing components described in this invention can function with comparable results when using pure distilled water (as in laboratory tests) or ordinary tap water (as in practical processing machines).

Example 16

A developer solution including, as a dye-forming developing agent (DFD), a compound of the formula:

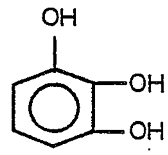

was formed from the following components:

*Solution A*

| | |
|---|---|
| Triethylene glycol | 100 ml |
| DFD | 10 g |

*Solution B*

| | |
|---|---|
| distilled water | 500 ml |
| trisodium phosphate | 60 g |
| hydroxylamine hydrochloride | 10 g |
| potassium bromide | 5 g |
| 5-nitroindazole (1% trigol solution) | 10 ml |

Solution B was added to solution A under stirring and the resulting solution was made up to 1000 ml with distilled water and the pH adjusted to 11.5.

The developer solution was used to process a sensitometrically exposed X-ray film as in Example 3, development time 1 minute at 32°C.

The densities obtained were measured and are reported in the following Table 15.

TABLE 15

| | Dmax | |
|---|---|---|
| Light | Silver and yellow dye image | Pale yellow dye image |
| White | 2.36 | 1.09 |
| Blue | 2.47 | 1.23 |

This Example displays the possibility of forming new colored image (pale yellow) using a dye-forming developing agent in a developer formulation containing components described in this invention.

Example 17

The developer solution of Example 2 with the exception that the black and white developer (metol) was omitted from the formulation was used to process a sensitometrically exposed X-ray emulsion having a 3.6 g/m$^2$ coating weight to give a blue-dye reinforced silver image. The processing was repeated using the developer solution containing metol. The densities resulting from the experiments were measured and are reported in the following Table 16.

TABLE 16

| Light | Dmax: Formulation containing metol | Dmax: Formulation not containing metol |
|---|---|---|
| White | 3.72 | 0.62 |
| Red | 4.19 | 0.74 |

This Example displays the role of a secondary black and white developer (the minor component as compared to the dye-forming developing agent) in the kinetic enhancement of the role of the image formation, as measured by Dmax.

Example 18

The formation of a blue-dye reinforced silver image using a two bath developer formulation where the dye-forming developing agent is present in one bath at a pH white does not allow it to reduce the latent image, while the second bath is of an alkaline pH capable of triggering the reduction reaction.

The following baths were prepared:

*Bath 1*

| | |
|---|---|
| triethylene glycol | 750 ml |
| 4-methoxy-1-naphthol | 10 g |
| distilled water | 250 ml |
| pH approximately 6 | |

*Bath 2*

| | |
|---|---|
| distilled water | 500 ml |
| sodium carbonate | 50 g |
| metol | 1 g |
| hydroxylamine HCl | 10 g |
| sodium sulfite | 10 g |
| potassium bromide | 5 g |
| diethanolamine | 50 g |
| pH adjusted to 11.2 with sodium hydroxide | |

An X-ray film having a silver coating weight of 3.6 g/m$^2$ was sensitometrically exposed and treated in bath 1 for 3 minutes at 40°C followed by treatment in bath 2 for 3 minutes at 32°C. For comparison a similar film was developed in a standard black and white developer (D19b). The densities obtained by the above processing were measured and are reported in the following Table 17.

TABLE 17

| Light | Dmax: Silver image | Dmax: Silver and blue dye image |
|---|---|---|
| White | 1.41 | 2.71 |
| Red | 1.51 | 3.33 |

The formation of dye-reinforcing silver image may be achieved by processing two consecutive baths, the first containing the dye-forming developing agent and the second triggering the development reaction.

Example 19

Dye-forming developer incorporated in an emulsion

An oil dispersion (A) of the trichloroacetyl derivative of MHN

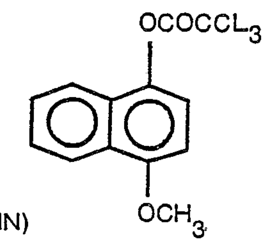

(TCAMHN)

was prepared by dissolving 32 g of TCAMHN in 64 g of dibutyl phthalate. The solution was then poured into 250 g of 10% gelatin and 1 to 2 ml of DOWFAX 241 anionic alkyl sulfonate surfactant and vigorously stirred with a high speed mixer.

The oil dispersion (A) was then incorporated into a photographic emulsion layer comprising:

| | |
|---|---|
| 1.3 $\mu$m silver iodobromide emulsion | 14 g (0.02,mol) |
| TCAMHN oil dispersion A) | 22 g (0.06 mol) |
| dimethylol urea 6% solution | 0.1 ml |
| resorcyl aldehyde 2% solution | 0.1 ml |
| pH | 5.5 |
| water to | 60 g |

The emulsion was coated onto a suitable film base at a silver coating weight of 28 mg/dm$^2$.

The coating was exposed for 0.1 second to blue filtered tungsten light through a neutral density continuous wedge.

The coating was processed in an alkali bath at pH 11.5 for 5 minutes (30 g $Na_2CO_3$/litre), then fixed and washed. A negative silver and blue dye image was obtained with a combined density of 0.68.

Example 20

A dye-forming developer (DFD) of the formula:

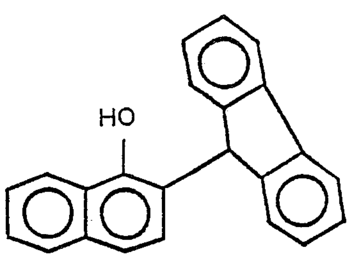

was incorporated into a silver halide 3M Type R X-ray emulsion in an oil dispersion in a similar manner to Example 16. 0.02 moles of silver halide 3M Type R X-ray emulsion which contained 7.7 g of 2% butyl phthalate oil dispersion of the above dye-forming developer, 0.2 g of metol (as an aqueous solution) and the usual ingredients known in the art of coating photographic layers, was hand coated using K bar No. 4 on a polyester base to give a photographic film of 2.3 g/m$^2$ silver. The coating, after being set, dried and hardened was exposed sensitometrically and processed in the following formulation:

| | |
|---|---|
| distilled water | 100 ml |
| sodium carbonate | 5 g |
| metol | 0.1 g |
| 5-nitroindazole (1% triethylene glycol solution) | 0.4 g |
| sodium hydroxide to pH = | 12.8 |

for a development time of 5 minutes at 20°C. The image produced was magenta (purple) in color.

As a reference, another sample film exposed in a similar manner but processed in a conventional Kodak D19b black and white developer (5 minutes at 20°C) to give a black silver image.

The densities of both images were measured using white and green light sources and the results are reported in the following Table 18.

TABLE 18

| | Dmax | |
|---|---|---|
| | white light | green light |
| Film and DFD | 1.06 | 1.19 |
| Reference | 1.04 | 1.04 |

The above Example shows the formation of a purple dye image when the above dye-forming developing agent is incorporated in the photographic emulsion and is activated by an aqueous activating solution.

Example 21

A photographic film was prepared as in Example 20 with the exception that the dye-forming developing agent (DFD) used was of the formula:

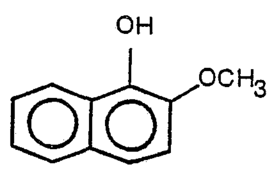

The film was exposed sensitometrically and processed in the following activating solution:

| | |
|---|---|
| distilled water | 500 ml |
| sodium carbonate | 25 g |
| potassium bromide | 0.5 g |
| metol | 0.05 g |

for a development time of 5 minutes at 20°C, to give a purple dye reinforced silver image. The densities were measured and are reported in the following Table 19.

TABLE 19

| Light | Dmax |
|---|---|
| White | 0.85 |
| Green | 1.05 |

This Example shows the use of another dye-forming developing agent capable of forming a purple-dye reinforced silver image.

Example 22

A photographic film was prepared as in Example 20 with the exception that the dye-forming developing agent used was of the formula:

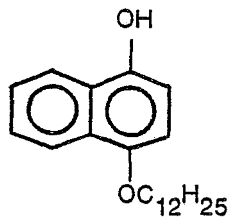

and was incorporated in ethylacetate in place of dibutylphthalate.

0.02 moles of silver 3M Type S photographic emulsion which contained 0.02 moles of the above dye-forming developing agent as a dispersion of ethylacetate solution, 0.2 g of metol (as an aqueous solution) and the usual ingredients known in the art of coating photographic layers, was hand coated using K bar No. 4 on a polyester base to give a photographic film of 2.0 g/m² silver. The coating after being set, dried and hardened was exposed sensitometrically and processed in the following activating solution for 5 minutes at 20°C:

| | |
|---|---|
| distilled water | 90ml |
| trisodium phosphate | 20 g |
| ethanol | 10 ml |

The densities were measured and are reported in the following Table 20.

TABLE 20

| Light | Dmax |
|---|---|
| White | 1.30 |
| Red | 1.54 |

This Example shows formation of a blue dye reinforced silver image when the dye-forming developing agent is incorporated in the photographic emulsion.

The dye-forming developer carried a ballasting 12-carbon chain to minimize its migration when processed in the alkaline activating solution.

The activation solution is an example of a simple aqueous alkaline solution.

The Dmax values display an amplification factor due to the presence of the blue dye.

Example 23

A photographic film was prepared as in Example 22 with the exception that the dye-forming developing agent (DFD) used was of the formula:

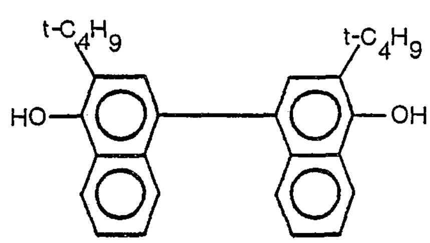

Upon processing a purple dye reinforced silver image was produced which exhibited the densities reported in the following Table 21.

TABLE 21

| Light | Dmax |
|---|---|
| White | 0.95 |
| Green | 1.20 |

This Example reflects the use of another dye-forming developing agent capable of forming a purple dye reinforced silver image.

Example 24

A photographic element was prepared as in Example 22 with the exception that the dye-forming developing agent used was of the formula:

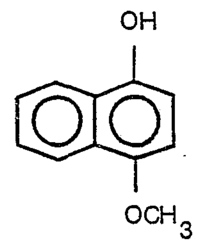

The sensitometrically photographic element was processed for 5 minutes at 20°C in aqueous alkaline activating solutions containing various amounts of polar organic solvent and/or a secondary black and white developer (metol) together with trisodium phosphate.

The image formed was a blue dye reinforced silver image. The densities obtained were measured and are reported in the following Table 22:

TABLE 22

| Activation solution | (1) | (2) | (3) | (4) |
|---|---|---|---|---|
| distilled water | 100 | 90 | 100 | 90 ml |
| trisodium phosphate | 6 | 6 | 6 | 6 g |
| metol | — | — | 0.1 | 0.1 g |
| methanol | — | 10 | 0 | 10 ml |

The rate of development of a photographic element containing a dye-forming developing agent in an oil dispersion can be controlled by the presence or absence of an organic polar solvent and/or a secondary black and white developer.

Example 25

A photographic element was prepared as in Example 24 but additionally including a yellow dye-forming developing agent of the formula:

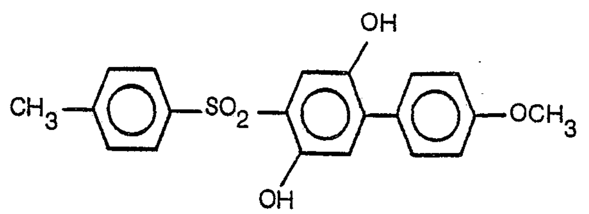

The ratio of blue:yellow dye-forming developing agent was 1:2. The sensitometrically exposed photographic element was processed for 5 minutes at 20°C in a solution comprising:

| | |
|---|---|
| distilled water | 90 ml |
| sodium carbonate | 6 g |
| methanol | 10 ml |

The density of the blackish image obtained was measured and is reported in the following Table 23.

TABLE 23

| Light | Dmax of silver and blue and yellow dye image |
|---|---|
| White | 1.48 |
| Red | 1.52 |
| Green | 1.53 |
| Blue | 1.38 |

The incorporation of one or more of the dye-forming developing agents in the photographic coating makes it possible to obtain a near to neutral colored final image.

Example 26

The photographic film of Example 23 was exposed sensitometrically and developed in the processing solution of Example 2 for 5 minutes at 20°C.

The image formed was a combination of a blue and purple-dye reinforced silver image which appeared as a black image. The densities were measured and are reported in the following Table 24.

TABLE 24

| Light | Dmax |
|---|---|
| White | 1.72 |
| Green | 1.74 |
| Blue | 1.70 |
| Red | 1.51 |

This Example displays the possibility of forming a silver image reinforced by two dyes of different colors from two dye-forming developing agents of different chemical structures and present in different media.

Example 27

The photographic film of Example 22 was exposed sensitometrically and processed in the developer solution of Example 7.

The image formed was a combination of a blue and yellow dye reinforced silver image which appeared as a black image.

As a reference the same photographic coating containing the blue dye-forming developing agent was processed in an alkaline activating solution (as described in Example 22) to give a blue-dye reinforced silver image. The densities were measured and the results are reported in the following Table 25.

TABLE 25

| Light | Dmax<br>Silver and blue dye image | Silver and blue and yellow dye image |
|---|---|---|
| White | 1.64 | 2.34 |
| Red | 1.63 | 2.40 |
| Green | 1.60 | 2.42 |
| Blue | 1.21 | 2.01 |

This Example displays the possibility of forming a silver image reinforced by two dyes of different colors from two dye-forming developing agents of different chemical structures and present in different media.

Some of the compounds included within the broad formulae of the present invention may be found in prior art photographic literature as additives to developing solutions or emulsions. For example, naphtholic color couplers for photographic emulsions are shown in U.S. Patents 2,474,293; 3,667,956 and 3,918,975 as well as Mees and James, *The Theory of the Photographic Process,* 3rd Ed., 1966, p. 392, Macmillian Co., N.Y. Developing agents such as p-aminophenols and their derivatives are also well known in the photographic art (e.g., Mees and James, *supra,* pp. 280—295, and Subject Index p. 575). These materials are not believed to be used in the compositions and process of the present invention, however.

For example, it is not believed that the prior art developers and couplers were used in solution with 1) a water-miscible polar organic solvent, 2) an alkanolamine or mono-aminocarboxylic acid, and 3) an hydroxylamine or hydroxamic acid. In some practices of the invention the presence of these known developing agents, e.g., with o-, m-, and especially p-amino groups, is not preferred. Many of these previously known developers for example do not form the preferred types of dyes. It is desirable that the dyes formed by oxidation of the developers in the practice of the present invention have extinction coefficients in excess of 1000 between 400 and 700 nm. These measurements may be generally taken in ethanol. Preferably the dyes have extinction coefficients in excess of 4000 and most preferably in excess of 7000 between 425 and 675 nm. The oxidation product of hydroquinone has an extinction coefficient of about 25 between 400 and 700 nm while compounds 37 and 38 have extinction coefficients of 3900 and 2500 in that region of the spectrum. Most oxidation products of black-and-white developers have similarly low extinction coefficients.

Compounds 62 and 93 have extinction coefficients of 100,000 or more, while compound 94 has an extinction coefficient of 50,000 and compounds 29 and 84 have extinction coefficients in excess of 15,000. Benzoquinone developers (both 1,4- and 1,2-benzoquinone) have extinction coefficients less than 50 while 1,4-naphthoquinone and 1,2-naphthoquinone have extinction coefficients of 2,500 and 2,000 respectively.
respectively.

When used within photographic emulsions, particularly in high temperature boiling water-imiscible organic solvent droplets, the dye forming developers of the present invention would tend to be used in concentrations far higher than color couplers. The most efficient couplers in use today are two-equivalent color couplers which require the reduction of two moles of silver for the production of one mole of dye. In the present invention, at least 1.50 or 1.75 moles of dye-forming developer are present in the oil-dispersed droplets for each mole of silver and preferably at least 2.0 moles of dye-forming developer are present for each mole of silver halide in the emulsion. Molar amounts up to 4, 5 and more (e.g., 10) times the amount of silver halide are, of course, quite useful. In formula 1, the normal coupling position for naphtholic couplers would be the 4-position. It is preferred in some cases, therefore, that the 4-position not be hydrogen, halogen, aryloxy, arylthio, and other conventional splitting-off or leaving groups.

The following are trademarks: RP-Xomat, Dowfax, 3M and Kodak.

**Claims**

1. A developer solution comprising an aqueous alkaline medium containing one or more dye-forming developing agents having the general formula:

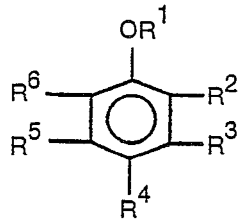

in which $R^1$ represents a hydrogen atom or hydrolysable group,

each of $R^2$ to $R^6$ is independently selected from hydrogen, halogen, an alkyl group, aryl group, alkoxy group, aryloxy group or amino group, hydroxy group, a thiol group or a thioether group, or two or more adjacent groups selected from $R^2$ to $R^6$ may represent the necessary atoms to complete one or more carbocyclic or heterocyclic ring groups, and at least two of the following components:

(1) a water-miscible polar organic solvent,

(2) at least one of an alkanolamine, substituted alkanolamine, and a monoamino carboxylic acid which may be substituted, and

(3) a nitrogen containing compound selected from the class of hydroxylamines, substituted hydroxylamines, aryl hydroxamic acid and alkyl hydroxamic acid which may be substituted.

2. A developer solution as claimed in Claim 1, which additionally includes a black and white developing agent.

3. A developer solution as claimed in Claim 2, which contains a total of up to 2 g/l of one or more black and white developing agents.

4. A developer solution as claimed in Claim 3, which contains said dye-forming developing agent and black and white developing agent in a weight ratio in the range of 15:1 to 5:1.

5. A developer solution as claimed in any preceding claim, which contains a total of 1 to 20 g/l of one or more of said dye-forming developing agents.

6. A developer solution as claimed in Claim 5, which contains a total of 7 to 12 g/l of one or more of said dye-forming developing agents.

7. A developer solution as claimed in any preceding claim, which contains up to 15% by weight of water-miscible polar organic solvent.

8. A developer solution as claimed in Claim 7, which contains 5 to 12% by weight of water-miscible polar organic solvent.

9. A developer solution as claimed in any preceding claim, which contains 50 to 150 g/l of component (2).

10. A developer solution as claimed in any preceding claim, which contains 1 to 15 g/l of component (3).

11. A developer solution as claimed in any preceding claim, which contains a polyhydroxy organic solvent as component (1).

12. The developer solution of any preceding Claim in which the dye-forming agent is represented by the formula:

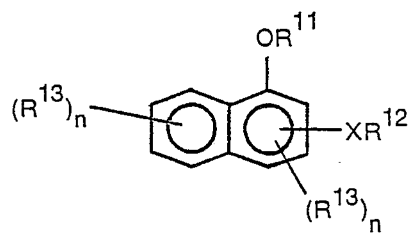

(2)

in which:

X is O, S or SE,

$XR^{12}$ can be in the 2 or 4 position,

$R^{11}$ is hydrogen or an alkali labile protecting group,

$R^{12}$ represents a ballasting group,

each $R^{13}$ independently represents a ring substituent selected from the group consisting of hydrogen, alkyl group, aryl group, hydroxy, alkoxy group, aryloxy group, amino group, alkylamino group, dialkylamino group, arylamino group, diarylamino group, carboxy, carboalkoxy group, carbonamido group, sulfonic acid, sulfonate, arylsulfonyl group, sulfoalkoxy group, sulfonamido group, and halide, and

n is an integer between 0 and 4.

13. The developer solution of any of Claims 1 to 12 in which the dye-forming developing agent is represented by any of the following formulae (3) to (14):

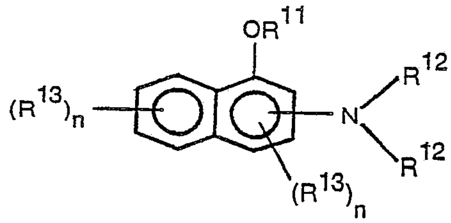

(3)

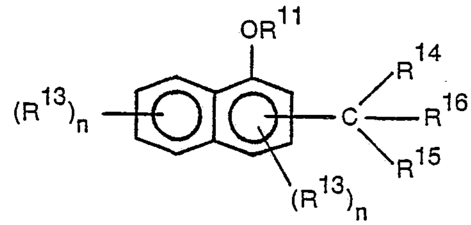

(4)

in which:

$R^{14}$ represents an alkyl group or hydrogen,

$R^{15}$ represents a hydrogen atom, alkyl group or an aromatic group, and

$R^{16}$ represents an aromatic group, said group being capable of activating the methine hydrogen of the developing agent when either $R^{14}$ or $R^{15}$ represents hydrogen, or

$R^{16}$ together with $R^{15}$ and their linking carbon atom represents the necessary atoms to complete a carbocyclic or heterocyclic ring group which is fused or linked to one or more aromatic rings other than the naphthalene nucleus, the group $—CR^{14}R^{15}R^{16}$ containing at least one aromatic ring;

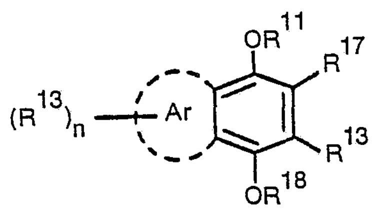

(5)

or

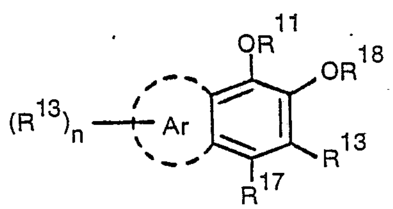

(6)

in which:

$R^{17}$ represents hydrogen, alkyl group, aryl group, alkylaryl group, alkyloxyaryl group, hydroxyaryl group, aminoaryl group, dialkylaminoaryl group, or forms a furan ring with the $\alpha$-hydroxy group,

$R^{18}$ represents hydrogen, alkyl group, arylalkyl group, alkoxyalkyl group, aminoalkyl group, quaternary ammonium alkyl group or alkyl sulfonate group,

and Ar represents a fused-on ring group comprising aromatic or heterocyclic ring groups;

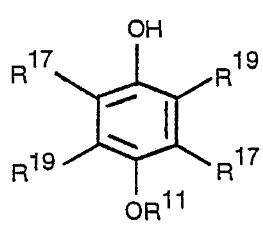

(7)

or

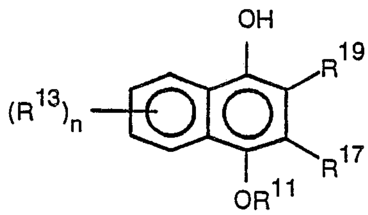

(8)

in which:

$R^{19}$ represents an aryl group or together with $R^{17}$ represents the necessary atoms to complete a fused on ring comprising aromatic or heterocyclic ring groups;

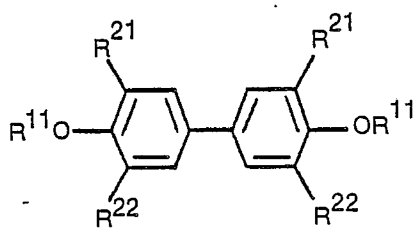

(9)

or

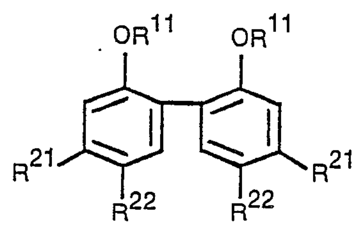

(10)

in which:

$R^{21}$ represents an alkyl group, alkoxy group, aryl group, or dialkylamino group, and

$R^{22}$ represents an alkyl group, aryl group, alkyoxy group, or dialkylamino group, or together with $R^{21}$ represents the necessary atoms to form an alicyclic, oxymethylene or aromatic ring group;

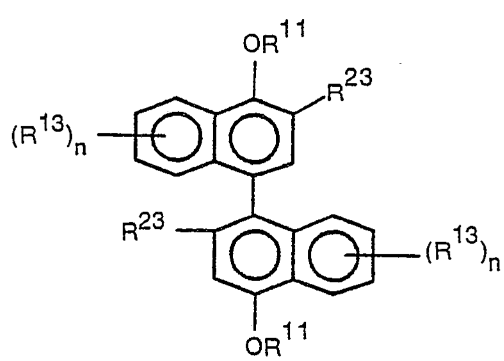

(11)

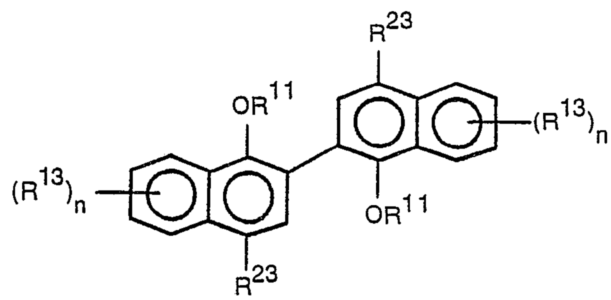

(12)

in which:

$R^{23}$ represents a hydrogen atom or an alkyl group, arylalkyl group, alkoxy group, aryloxy group, alkylaryloxy group, aryl group, alkylaryl group, alkoxyaryl group, hydroxyaryl group, aminoaryl group, alkylaminoaryl, dialkylaminoaryl group, carboalkoxy group, carboaryloxy group, carboamido group, alkylamino group, arylamino group, diarylamino group or N-heterocyclic group and

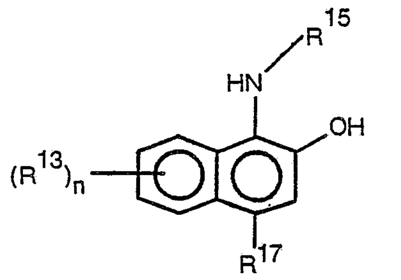

(13)

or

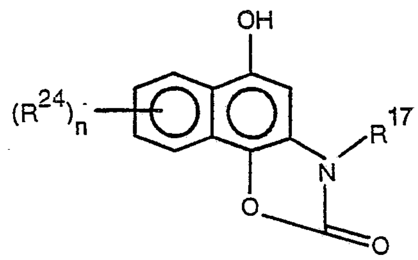

(14)

in which:

$R^{24}$ represents a hydrogen atom, an alkyl group, alkoxy group, hydroxy amino group, alkylamino group, dialkylamino group, N-heterocyclic group or aryl group or forms a fused aromatic or heterocyclic ring group,

wherein in all the above formulae $R^{11}$ is hydrogen or an alkali labile group,

$R^{12}$ is a ballasting group or the atoms necessary to form a heterocyclic ring group,

$R^{13}$ is selected from the group consisting of hydrogen, alkyl groups, aryl groups, hydroxy, alkoxy groups, aryloxy groups, amino groups, alkylamino groups, dialkylamino groups, arylamino groups, diarylamino groups, carboxy, carboalkoxy groups, carboamido groups, sulfonic acid, sulfonate, arylsulfonyl groups, sulfoalkoxy groups, sulfoamido groups, halide,

n is an integer selected from 0, 1, 2, 3, and 4, and groups

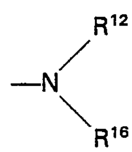

and

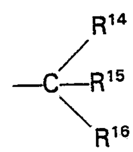

may be located only in the 2 and 4 positions.

14. A photographic development process in which an exposed halide photographic film is developed in an alkaline environment in the presence of one or more dye-forming developing agents having the general formula:

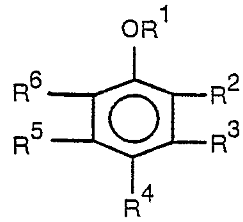

in which $R^1$ is hydrogen or a hydrolyzable group and $R^2$ to $R^6$ are independently selected from hydrogen, halogen, an alkyl group, aryl group, alkoxy group, aryloxy group or amino group, hydroxy group, a thiol group or a thioether group, or two or more adjacent groups selected from $R^2$ to $R^6$ may represent the necessary atoms to complete one or more carbocyclic or heterocyclic ring groups, and at least two of the following components:

(1) a water-miscible polar organic solvent,

(2) at least one of an alkanolamine, substituted alkanolamine, and a monoamino carboxylic acid which may be substituted, and

(3) at least one nitrogen containing compound selected from the group consisting of a hydroxylamine, substituted hydroxylamine, an aryl hydroxamic acid or alkyl hydroxamic acid, any of which may be substituted.

15. A process as claimed in Claim 14 in which the film is developed in the presence of a black and white developing agent.

16. A photographic emulsion comprising a hydrophilic colloid having dispersed therein oil droplets containing photographic silver halide grains and a dye forming developer of the formula:

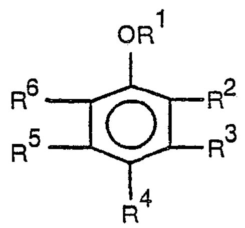

in which $R^1$ represents a hydrogen atom or hydrolyzable group,

each of $R^2$ to $R^6$ is independently selected from hydrogen, halogen, an alkyl group, aryl group, alkoxy group, aryloxy group or amino group, hydroxy group, a thiol group or a thioether group, or two or more adjacent groups selected from $R^2$ to $R^6$ may represent the necessary atoms to complete one or more carbocyclic or heterocyclic ring groups, and at least two of the following components:

(1) a water-miscible polar organic solvent,

(2) at least one of an alkanolamine, substituted alkanolamine, and a monoamino carboxylic acid which may be substituted, and

(3) a nitrogen containing compound selected from the class of hydroxylamines, substituted hydroxylamines, aryl hydroxamic acid and alkyl hydroxamic acid which may be substituted.

17. The photographic emulsion of Claim 16 wherein said dye forming developer has any of the following formulae (3) to (14):

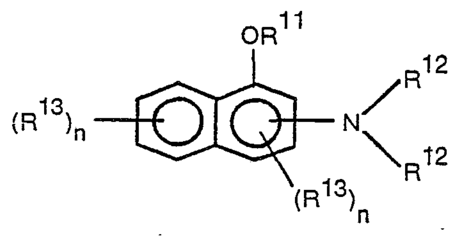

(3)

or

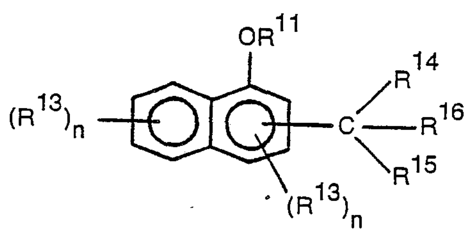

(4).

in which:

$R^{14}$ represents an alkyl group or hydrogen,

$R^{15}$ represents a hydrogen atom, alkyl group or an aromatic group, and

$R^{16}$ represents an aromatic group, said group being capable of activating the methine hydrogen of the developing agent when either $R^{14}$ or $R^{15}$ represents hydrogen, or

$R^{16}$ together with $R^{15}$ and their linking carbon atom represents the necessary atoms to complete a carbocyclic or heterocyclic ring group which is fused or linked to one or more aromatic rings other than the naphthalene nucleus, the group $-CR^{14}R^{15}R^{16}$ containing at least one aromatic ring;

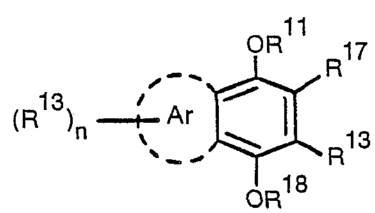

(5)

or

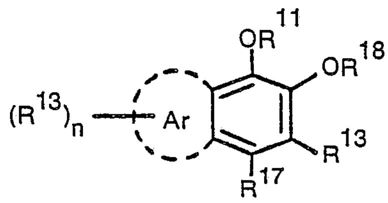

(6)

in which:

$R^{17}$ represents hydrogen, alkyl group, aryl group, alkylaryl group, alkyloxyaryl group, hydroxyaryl group, aminoaryl group, dialkylaminoaryl group, or forms a furan ring with the $\alpha$-hydroxy group,

$R^{18}$ represents hydrogen, alkyl group, arylalkyl group, alkoxyalkyl group, aminoalkyl group, quaternary ammonium alkyl group or alkyl sulfonate group,

and Ar represents a fused-on ring group comprising aromatic or heterocyclic ring groups;

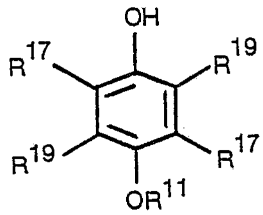

(7)

or

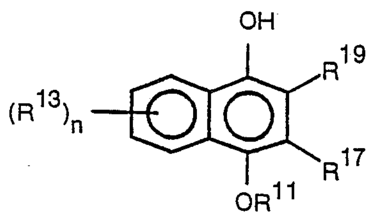

(8)

in which:

$R^{19}$ represents an aryl group or together with $R^{17}$ represents the necessary atoms to complete a fused on ring comprising aromatic or heterocyclic ring groups;

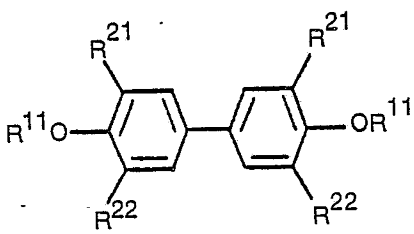

(9)

or

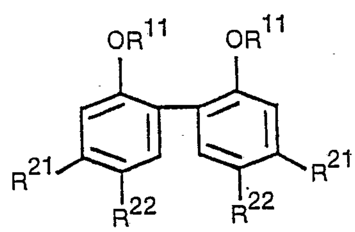

(10)

in which:

$R^{21}$ represents an alkyl group, alkoxy group, aryl group, or dialkylamino group, and

$R^{22}$ represents an alkyl group, aryl group, alkyoxy group, or dialkylamino group, or together with $R^{21}$ represents the necessary atoms to form an alicyclic, oxymethylene or aromatic ring group;

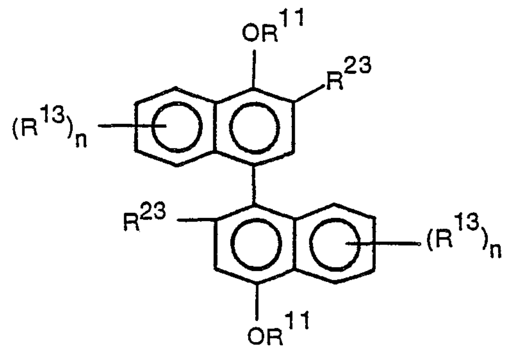

(11)

or

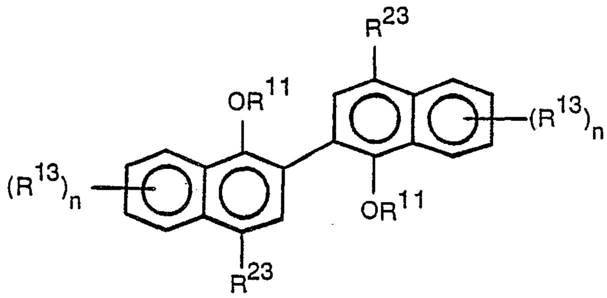

(12)

in which :

$R^{23}$ represents a hydrogen atom or an alkyl group, arylalkyl group, alkoxy group, aryloxy group, alkylaryloxy group, aryl group, alkylaryl group, alkoxyaryl group, hydroxyaryl group, aminoaryl group, alkylaminoaryl, dialkylaminoaryl group, carboalkoxy group, carboaryloxy group, carboamido group, alkylamino group, arylamino group, diarylamino group or N-heterocyclic group and

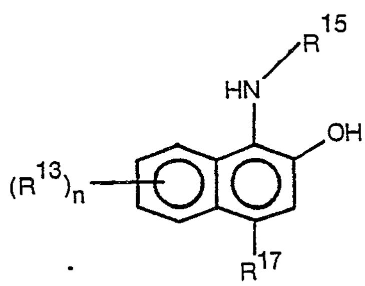

(13)

or

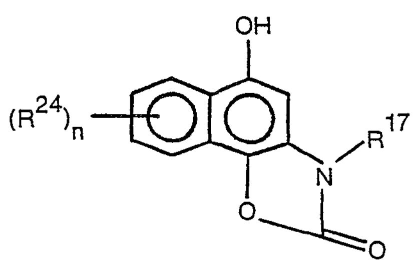

(14)

in which:

$R^{24}$ represents a hydrogen atom, an alkyl group, alkoxy group, hydroxy amino group, alkylamino group, dialkylamino group, N-heterocyclic group or aryl group or forms a fused aromatic or heterocyclic ring group,

wherein in all the above formulae $R^{11}$ is hydrogen or an alkali labile group,

$R^{12}$ is a ballasting group or the atoms necessary to form a heterocyclic ring group,

$R^{13}$ is selected from the group consisting of hydrogen, alkyl groups, aryl groups, hydroxy, alkoxy groups, aryloxy groups, amino groups, alkylamino groups, dialkylamino groups, arylamino groups, diarylamino groups, carboxy, carboalkoxy groups, carboamido groups, sulfonic acid, sulfonate, arylsulfonyl groups, sulfoalkoxy groups, sulfoamido groups, halide,

n is an integer selected from 0, 1, 2, 3, and 4, and groups

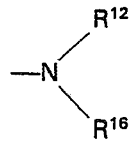

and

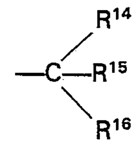

may be located only in the 2 and 4 positions.

18. The emulsion of Claims 16 or 17 wherein said dye forming developer is present in a ratio of at least 2.0 moles of developer per mole of silver halide.

**Patentansprüche**

1. Eine Entwicklerlösung, umfassend ein Wäßriges alkalisches Medium, das mindestens einen farbstoffbildenden Entwickler der allgemeinen Formel

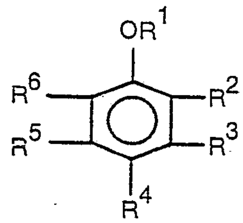

in der $R^1$ ein Wasserstoffatom oder einen hydrolysierbaren Rest darstellt,
jeder der Reste $R^2$ bis $R^6$ unabhängig Wasserstoff, Halogen, einen Alkyl-, Aryl-, Alkoxy- oder Aryloxyrest oder eine Amino-, Hydroxy-, Thiol- oder Thioäthergruppe bedeutet oder zwei oder mehrere benachbarte Reste $R^2$ bis $R^6$ die zur Vervollständigung von mindestens einem carbocyclischen oder heterocyclischen Ring notwendigen Atome darstellen, und mindestens zwei der folgenden Komponenten enthält:

(1) ein mit Wasser mischbares polares organisches Lösungsmittel,

(2) mindestens ein Alkanolamin, substituiertes Alkanolamin oder eine Monoaminocarbonsäure, welche substituiert sein kann, und

(3) eine stickstoffhaltige Verbindung aus der Gruppe der Hydroxylamine, substituierten Hydroxylamine, Arylhydroxamsäuren und Alkylhydroxamsäuren, welche substituiert sein können.

2. Eine Entwicklerlösung nach Anspruch 1, welche zusätzlich einen Schwarz-Weiß-Entwickler enthält.

3. Eine Entwicklerlösung nach Anspruch 2, welche insgesamt bis zu 2 g/Liter mindestens eines Schwarz-Weiß-Entwicklers enthält.

4. Eine Entwicklerlösung nach Anspruch 3, welche den farbbildenden Entwickler und den Schwarz-Weiß-Entwickler in einem Gewichtsverhältnis im Bereich von 15:1 bis 5:1 enthält.

5. Eine Entwicklerlösung nach einem der vorangehenden Ansprüche, welche insgesamt 1 bis 20 g/Liter mindestens eines der genannten farbbildenden Entwickler enthält.

6. Eine Entwicklerlösung nach Anspruch 5, welche insgesamt 7 bis 12 g/Liter mindestens eines der genannten farbbildenden Entwickler enthält.

7. Eine Entwicklerlösung nach einem der vorangehenden Ansprüche, welche bis zu 15 Gew.-% mit Wasser mischbares polares organisches Lösungsmittel enthält.

8. Eine Entwicklerlösung nach Anspruch 7, welche 5 bis 12 Gew.-% mit Wasser mischbares polares organisches Lösungsmittel enthält.

9. Eine Entwicklerlösung nach einem der vorangehenden Ansprüche, welche 50 bis 150 g/Liter der Komponente (2) enthält.

10. Eine Entwicklerlösung nach einem der vorangehenden Ansprüche, welche 1 bis 15 g/Liter der Komponente (3) enthält.

11. Eine Entwicklerlösung nach einem der vorangehenden Ansprüche, welche ein organisches Polyhydroxy-Lösungsmittel als Komponente (1) enthält.

12. Entwicklerlösung nach einem vorangehenden Anspruch, in der der farbbildende Entwickler durch die Formel

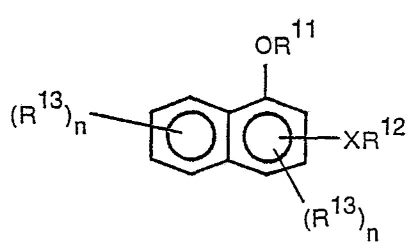

(2)

dargestellt wird, in der

X, O, S oder Se bedeutet,

$XR^{12}$ sich in 2- oder 4-Stellung befinden kann,

$R^{11}$ Wasserstoff oder eine alkalilabile Schutzgruppe darstellt,

$R^{12}$ eine Ballastgruppe bedeutet,

alle Reste $R^{13}$ unabhängig Ringsubstituenten aus der Gruppe Wasserstoff, Alkyl- Aryl-, Hydroxy-, Alkoxy-, Aryloxy-, Amino-, Alkylamino-, Dialkylamino-, Arylamino-, Diarylamino-, Carboxy-, Carboalkoxy-, Carbonamido-, Sulfonsäure-, Sulfonat-, Arylsulfonyl-, Sulfoalkoxy- und Sulfonamidoreste sowie Halogenatome darstellen und

n eine ganze Zahl zwischen 0 und 4 ist.

13. Entwicklerlösung nach einem der Ansprüche 1 bis 12, in der der farbbildende Entwickler durch eine der folgenden Formeln (3) bis (14) wiedergegeben wird:

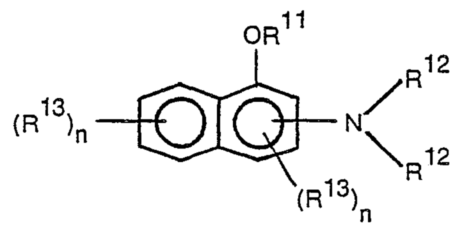

(3)

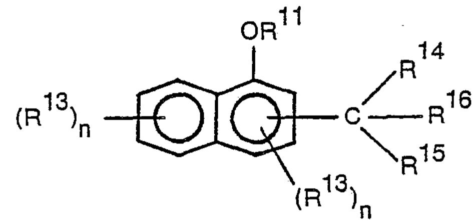

(4)

in denen

$R^{14}$ einen Alkylrest oder ein Wasserstoffatom bedeutet,

$R^{15}$ ein Wasserstoffatom, einen Alkylrest oder einen aromatischen Rest darstellt, und

$R^{16}$ einen aromatischen Rest bedeutet, wobei dieser Rest in der Lage ist, das Methin-Wasserstoffatom des Entwicklers zu aktivieren, wenn entweder $R^{14}$ oder $R^{15}$ ein Wasserstoffatom bedeutet, oder

$R^{16}$ zusammen mit $R^{15}$ und dem sie verbindenden Kohlenstoffatom die zur Vervollständigung eines carbocyclischen oder heterocyclischen Rings, welcher an einen oder mehrere aromatische Ringe außer dem Naphthalinkern ankondensiert oder damit verbunden ist, notwenigen Atome bedeutet, wobei der Rest $—CR^{14}R^{15}R^{16}$ mindestens einen aromatischen Ring enthält;

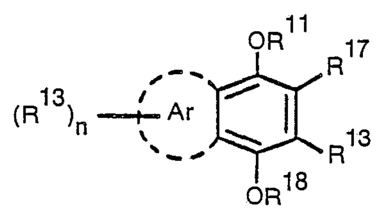

(5)

oder

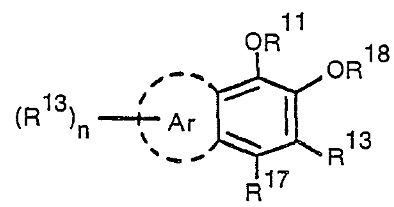

(6)

in denen

$R^{17}$ ein Wasserstoffatom, einen Alkyl-, Aryl-, Alkylaryl-, Alkyloxyaryl-, Hydroxyaryl-, Aminoaryl- oder Dialkylaminoarylrest bedeutet oder mit der $\alpha$-Hydroxygruppe einen Furanring bildet,

$R^{18}$ ein Wasserstoffatom, einen Alkyl-, Arylalkyl-, Alkoxyalkyl-, Aminoalkyl-, quaternären Ammoniumalkyl-, oder Alkylsulfonatrest darstellt, und

Ar einen ankondensierten Ring darstellt, der aromatische oder heterocyclische Ringgruppen umfaßt;

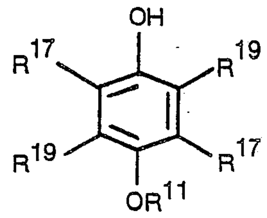

(7)

oder

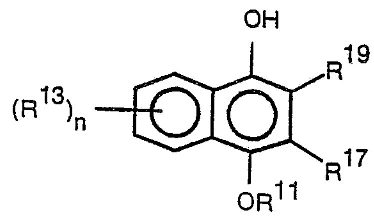

(8)

in denen

$R^{19}$ einen Arylrest darstellt oder zusammen mit $R^{17}$ die zur Vervollständigung eines ankondensierten, aromatische oder heterocyclische Ringgruppen enthaltenden Rings notwendigen Atome bedeutet;

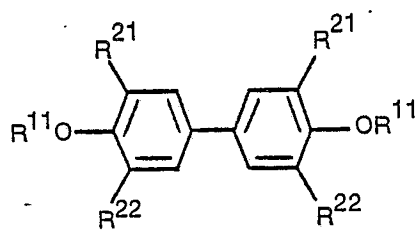

(9)

oder

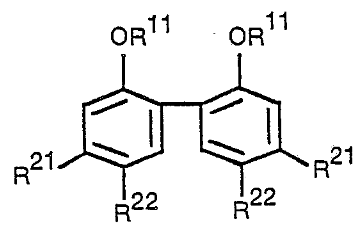

(10)

in denen

$R^{21}$ einen Alkyl-, Alkoxy-, Aryl- oder Dialkylaminorest darstellt, und

$R^{22}$ einen Alkyl-, Aryl-, Alkyloxy- oder Dialkylaminorest bedeutet oder zusammen mit $R^{21}$ die für einen alicyclischen, Oxymethylen- oder aromatischen Rest erforderlichen Atome bedeutet;

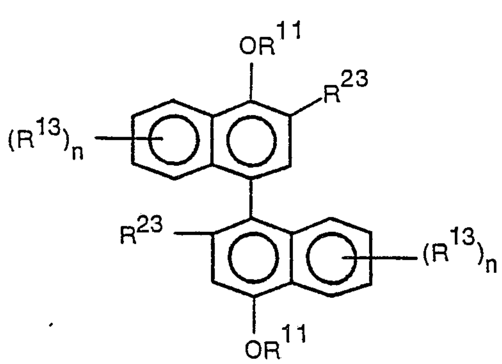

(11)

oder

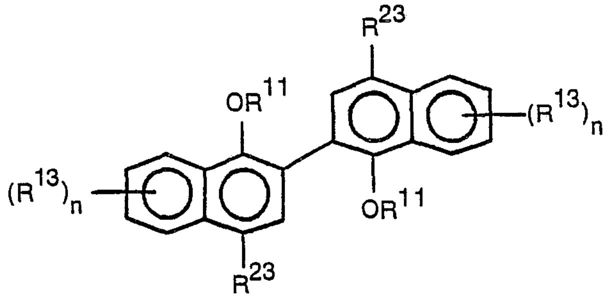

(12)

in denen

$R^{23}$ eine Wasserstoffatom oder einen Alkyl-, Arylalkyl-, Alkoxy-, Aryloxy-, Alkylaryloxy-, Aryl-, Alkylaryl-, Alkoxyaryl-, Hydroxyaryl-, Aminoaryl-, Alkylaminoaryl-, Dialkylaminoaryl-, Carboalkoxy-, Carboaryloxy-, Carboamido-, Alkylamino-, Arylamino-, Diarylamino- oder N-heterocyclischen Rest bedeutet; und

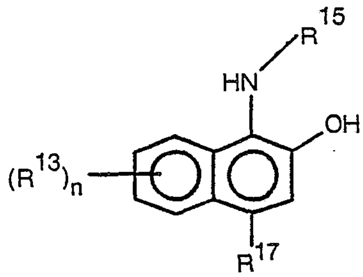

(13)

oder

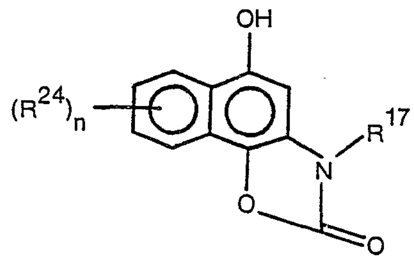

(14)

in denen

ein Wasserstoffatom, einen Alkyl-, Alkoxy-, Hydroxyamino-, Alkylamino-, Dialkylamino-, N-heterocyclischen oder Arylrest bedeutet oder einen ankondensierten aromatischen oder heterocyclischen Ring bildet,

wobei in allen vorstehenden Formeln $R^{11}$ ein Wasserstoffatom oder eine säurelabile Gruppe ist,

$R^{12}$ eine Ballastgruppe oder die für einen heterocyclischen Ring erforderlichen Atome darstellt,

$R^{13}$ ein Wasserstoffatom, einen Alkyl-, Aryl-, Hydroxy-, Alkoxy-, Aryloxy-, Amino-, Alkylamino-, Dialkylamino-, Arylamino-, Diarylamino-, Carboxy-, Carboalkoxy-, Carboamido-, Sulfonsäure-, Sulfonat-, Arylsulfonyl-, Sulfoalkoxy- oder Sulfoamidorest oder ein Halogenatom darstellt,

n eine ganze Zahl im Wert von 0, 1, 2, 3 oder 4 ist und die Reste

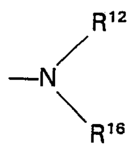 und 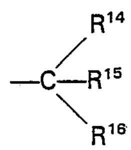

sich nur in 2- und 4-Stellung befinden können.

14. Eine photographisches Entwicklungsverfahren, bei dem ein belichteter photographischer Silberhalogenidfilm in einer alkalischen Umgebung in Gegenwart von mindestens einem farbstoffbildenden Entwickler der allgemeinen Formel

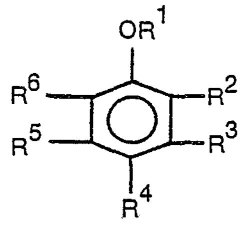

in der $R^1$ ein Wasserstoffatom oder einen hydrolysierbaren Rest darstellt, jeder der Reste $R^2$ bis $R^6$ unabhängig Wasserstoff, Halogen, einen Alkyl-, Aryl-, Alkoxy- oder Aryloxyrest oder eine Amino-, Hydroxy-, Thiol- oder Thioäthergruppe bedeutet oder zwei oder mehrere benachbarte Reste $R^2$ bis $R^6$ die zur Vervollständigung von mindestens einem carbocyclischen oder heterocyclischen Ring notwendigen Atome darstellen, und von mindestens zwei der folgenden Komponenten

(1) ein mit Wasser mischbares polares organisches Lösungsmittel,

(2) mindestens ein Alkanolamin, substituiertes Alkanolamin oder eine Monoaminocarbonsäure, welche substituiert sein kann, und

(3) mindestens eine stickstoffhaltige Verbindung aus der Gruppe der Hydroxylamine, substituierten Hydroxylamine, Arylhydroxamsäuren und Alkylhydroxamsäuren, welche alle substituiert sein können,

entwickelt wird.

15. Eine Verfahren nach Anspruch 14, bei dem der Film in Gegenwart eines Schwarz-Weiß-Entwicklers entwickelt wird.

16. Eine photographische Emulsion, umfassend ein hydrophiles Kolloid, das Öltröpfchen dispergiert enthält, welche photographische Silberhalogenidkörner und einen farbstoffbildenden Entwickler der allgemeinen Formel

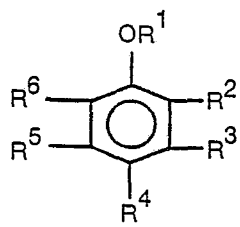

in der $R^1$ ein Wasserstoffatom oder einen hydrolysierbaren Rest darstellt, jeder der Reste $R^2$ bis $R^6$ unabhängig Wasserstoff, Halogen, einen Alkyl-, Aryl-, Alkoxy- oder Aryloxyrest oder eine Amino-, Hydroxy-, Thiol- oder Thioäthergruppe bedeutet oder zwei oder mehrere benachbarte Reste $R^2$ bis $R^6$ die zur Vervollständigung von mindestens einem carbocyclischen oder heterocyclischen Ring notwendigen Atome darstellen, und mindestens zwei der folgenden Komponenten enthält:

(1) ein mit Wasser mischbares polares organisches Lösungsmittel,

(2) mindestens ein Alkanolamin, substituiertes Alkanolamin oder eine Monoaminocarbonsäure, welche substituiert sein kann, und

(3) eine stickstoffhaltige Verbindung aus der Gruppe der Hydroxylamine, substituierten Hydroxylamine, Arylhydroxamsäuren und Alkylhydroxamsäure, welche substituiert sein können.

17. Photographische Emulsion nach Anspruch 16, in der der farbbildende Entwickler durch eine der folgenden Formeln (3) bis (14) wiedergegeben wird:

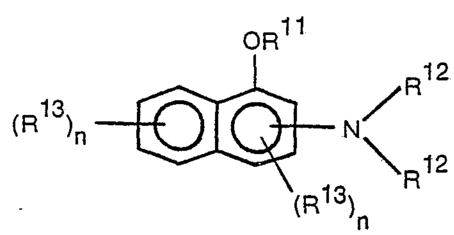

(3)

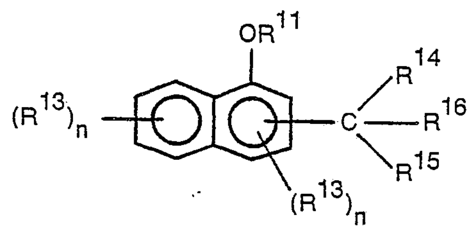

(4)

in denen

$R^{14}$ einen Alkylrest oder ein Wasserstoffatom bedeutet,

$R^{15}$ ein Wasserstoffatom, einen alkylrest oder einen aromatischen Rest darstellt, und

$R^{16}$ einen aromatischen Rest bedeutet, wobei dieser Rest in der Lage ist, das Methin-Wasserstoffatom des Entwicklers zu aktivieren, wenn entweder $R^{14}$ oder $R^{15}$ ein Wasserstoffatom bedeutet, oder

$R^{16}$ zusammen mit $R^{15}$ und dem sie verbindenden Kohlenstoffatom die zur Vervollständigung eines carbocyclischen oder heterocyclischen Rings, welcher an einen oder mehrere aromatische Ringe außer dem Naphthalinkern ankondensiert oder damit verbunden ist, notwenigen Atome bedeutet, wobei der Rest $—CR^{14}R^{15}R^{16}$ mindestens einen aromatischen Ring enthält;

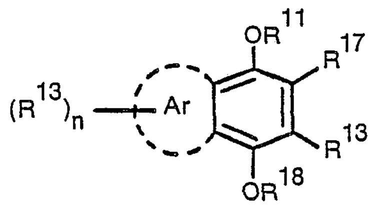

(5)

oder

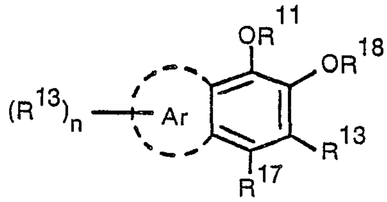

(6)

in denen

$R^{17}$ ein Wasserstoffatom, einen Alkyl-, Aryl-, Alkylaryl-, Alkyloxyaryl-, Hydroxyaryl-, Aminoaryl- oder Dialkylaminoarylrest bedeutet oder mit der $\alpha$-Hydroxygruppe einen Furanring bildet,

$R^{18}$ ein Wasserstoffatom, einen Alkyl-, Arylalkyl-, Alkoxyalkyl-, Aminoalkyl-, quaternären Ammoniumalkyl-, oder Alkylsulfonatrest darstellt, und

Ar einen ankondensierten Ring darstellt, der aromatische oder heterocyclische Ringgruppen umfaßt;

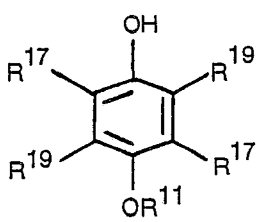

(7)

oder

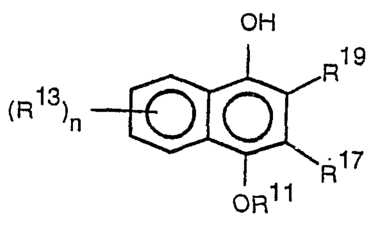

(8)

in denen

$R^{19}$ einen Arylrest darstellt oder zusammen mit $R^{17}$ die zur Vervollständigung eines ankondensierten, aromatische oder heterocyclische Ringgruppen enthaltenden Rings notwendigen Atome bedeutet;

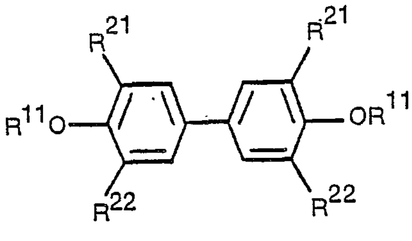

(9)

oder

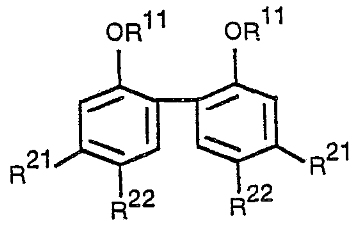

(10)

in denen
$R^{21}$ einen Alkyl-, Alkoxy-, Aryl- oder Dialkylaminorest darstellt, und
$R^{22}$ einen Alkyl-, Aryl-, Alkyloxy- oder Dialkylaminorest bedeutet oder zusammen mit $R^{21}$ die für einen alicyclischen, Oxymethylen- oder aromatischen Rest erforderlichen Atome bedeutet;

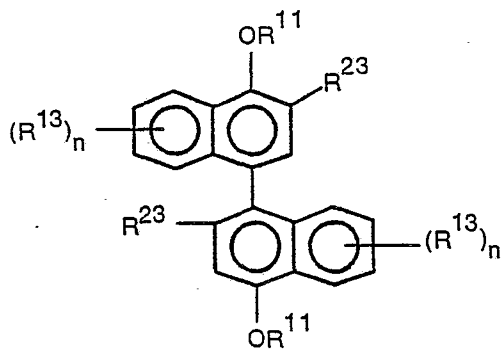

(11)

oder

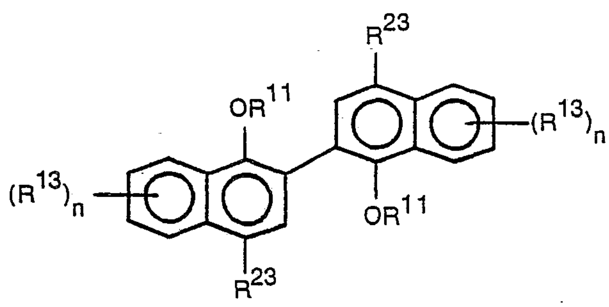

(12)

in denen
$R^{23}$ eine Wasserstoffatom oder einen Alkyl-, Arylalkyl-, Alkoxy-, Aryloxy-, Alkylaryloxy-, Aryl-, Alkylaryl-, Alkoxyaryl-, Hydroxyaryl-, Aminoaryl-, Alkylaminoaryl-, Dialkylaminoaryl-, Carboalkoxy-, Carboaryloxy-, Carboamido-, Alkylamino-, Arylamino-, Diarylamino- oder N-heterocyclischen Rest bedeutet; und

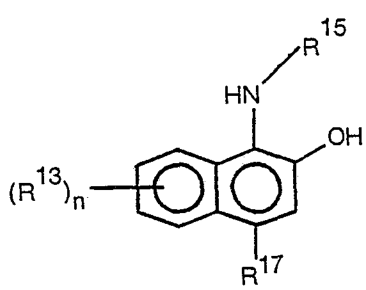

(13)

oder

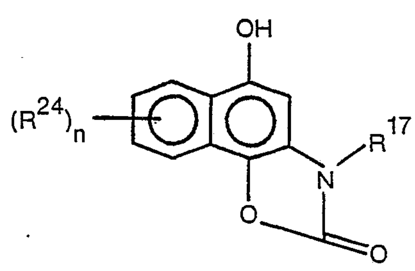

(14)

in denen

ein Wasserstoffatom, einen Alkyl-, Alkoxy-, Hydroxyamino-, Alkylamino-, Dialkylamino-, N-heterocyclischen oder Arylrest bedeutet oder einen ankondensierten aromatischen oder heterocyclischen Ring bildet,

wobei in allen vorstehenden Formeln $R^{11}$ ein Wasserstoffatom oder eine säurelabile Gruppe ist, $R^{12}$ eine Ballastgruppe oder die für einen heterocyclischen Ring erforderlichen Atome darstellt,

$R^{13}$ ein Wasserstoffatom, einen Alkyl-, Aryl-, Hydroxy-, Alkoxy-, Aryloxy-, Amino-, alkylamino-, Dialkylamino-, Arylamino-, Diarylamino-, Carboxy-, Carboalkoxy-, Carboamido-, Sulfonsäure-, Sulfonat-, Arylsulfonyl-, Sulfoalkoxy- oder Sulfoamidorest oder ein Halogenatom darstellt,

n eine ganze Zahl im Wert von 0, 1, 2, 3 oder 4 ist und die Reste

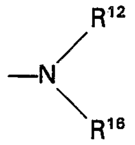 und 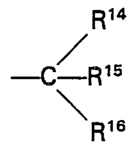

sich nur in 2- und 4-Stellung befinden können.

18. Emulsion nach den Ansprüchen 16 oder 17, in der der farbstoffbildende Entwickler in einem Verhältnis von mindestens 2,0 Mol Entwickler pro Mol Silberhalogenid vorhanden ist.

**Revendications**

1. Une solution révélatrice comprenant un milieu alcalin aqueux contenant un ou plusieurs agents révélateurs formant une matière colorante répondant à la formule générale:

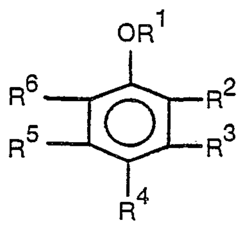

dans laquelle $R^1$ représente un atome d'hydrogène ou un groupe hydrolysable,

chacun de $R^2$ et $R^6$ est indépendamment choisi parmi un hydrogène, un halogène, un groupe alkyle, un groupe aryle, un groupe alcoxy, un groupe aryloxy ou un groupe amino, un groupe hydroxy, un groupe thiol ou un groupe thioéther, ou deux ou plusieurs groupes adjacents choisis parmi $R^2$ à $R^6$ peuvent représenter les atomes nécessaires pour réaliser un ou plusieurs groupes cycliques carbocycliques ou hétérocycliques, et au moins deux des composants suivants:

(1) un solvant organique polaire miscible à l'eau,

(2) au moins une alcanolamine, une alcanolamine substituée et un acide monoaminocarboxylique pouvant être substitué, et

(3) un composé azoté choisi dans la classe des hydroxylamines, des hydroxylamines substituées, des acides arylhydroxamiques et des acides alkylhydroxamiques pouvant être substitués.

2. Une solution révélatrice comme revendiquée dans la revendication 1, qui contient de plus un agent révélateur en noir et blanc.

3. Une solution révélatrice comme revendiquée dans la revendication 2 qui contient au total jusqu'à 2 g/l d'un ou plusieurs agents révélateurs en noir et blanc.

4. Une solution révélatrice comme revendiquée dans la revendication 3 qui contient ledit agent révélateur formant une matière colorante et un agent révélateur en noir et blanc dans un rapport pondéral compris dans la gamme de 15/1 à 5/1.

5. Une solution révélatrice comme revendiquée dans l'une quelconque des revendications précédentes, qui contient au total 1 à 20 g/l d'un ou plusieurs desdits agents révélateurs formant une matière colorante.

6. Une solution révélatrice comme revendiquée dans la revendication 5 qui contient au total 7 à 12 g/l d'un ou plusieurs desdits agents révélateurs formant une matière colorante.

7. Une solution révélatrice comme revendiquée dans l'une quelconque des revendications précédentes qui contient jusqu'à 15% en poids de solvant organique polaire miscible à l'eau.

8. Une solution révélatrice comme revendiquée dans la revendication 7 qui contient 5 à 12% en poids de solvant organique polaire miscible à l'eau.

9. Une solution révélatrice comme revendiquée dans l'une quelconque des revendications précédentes qui contient 50 à 150 g/l du composant (2).

10. Une solution révélatrice comme revendiquée dans l'une quelconque des revendications précédentes qui contient 1 à 15 g/l du composant (3).

11. Une solution révélatrice comme revendiquée dans l'une quelconque des revendications précédentes qui contient un solvant organique polyhydroxylé comme composant (1).

12. La solution révélatrice de l'une quelconque des revendications précédentes dans laquelle l'agent révélateur formant une matière colorante est représenté par la formule:

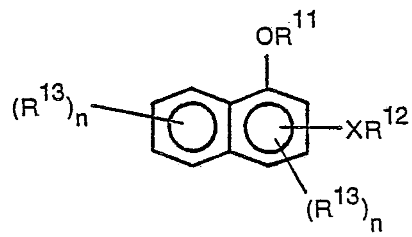

dans laquelle:

X est O, S ou Se,

$XR^{12}$ peut être en position 2 ou 4,

$R^{11}$ est un hydrogène ou un groupe protecteur labile en conditions alcalines,

$R^{12}$ représente un groupe de lestage,

chaque $R^{13}$ représente indépendamment un substituant du cycle choisi dans le groupe constitué par un hydrogène, un groupe alkyle, un groupe aryle, un hydroxy, un groupe alcoxy, un groupe aryloxy, un groupe amino, un groupe alkylamino, un groupe dialkylamino, un groupe arylamino, un groupe diarylamino, un carboxy, un groupe carbalcoxy, un groupe carbonamido, un acide sulfonique, un sulfonate, un groupe arylsulfonyle, un groupe sulfalcoxy, un groupe sulfonamido et un halogénure, et

n est un entier entre 0 et 4.

13. La solution révélatrice de l'une quelconque des revendications 1 à 12 dans laquelle l'agent révélateur formant une matière colorante est représenté par l'une quelconque des formules (3) à (14) suivantes:

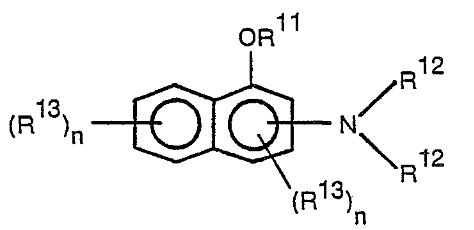

(3)

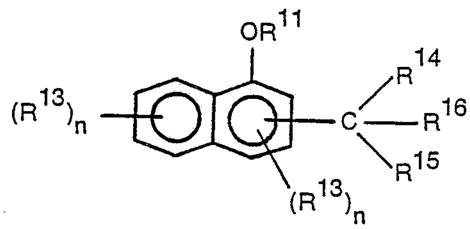

(4)

où

$R^{14}$ représente un groupe alkyle ou un hydrogène,

$R^{15}$ représente un atome d'hydrogène, un groupe alkyle ou un groupe aromatique, et

$R^{16}$ représente un groupe aromatique, ledit groupe étant capable d'activer l'hydrogène méthinique de l'agent révélateur lorsque $R^{14}$ ou $R^{15}$ représente un hydrogène, ou

$R^{16}$ avec $R^{15}$ et leurs atome de carbone de liaison représentent les atomes nécessaires pour réaliser un groupe cyclique carbocyclique ou hétérocyclique qui est condensé ou uni à un ou plusieurs cycles aromatiques autres que le noyau naphtalène, le groupe $—CR^{14}R^{15}R^{16}$ contenant au moins un cycle aromatique;

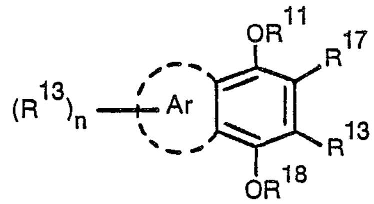

(5)

ou

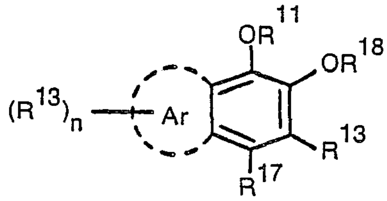

(6)

ou:

$R^{17}$ représente un hydrogène, un groupe alkyle, un groupe aryle, un groupe alkylaryle, un groupe alcoxyaryle, un groupe hydroxyaryle, un groupe aminoaryle, un groupe dialkylaminoaryle ou forme un cycle furanne avec le groupe $\alpha$-hydroxy,

$R^{18}$ représente un hydrogène, un groupe alkyle, un groupe arylalkyle, un groupe alcoxyalkyle, un groupe aminoalkyle, un groupe alkylammonium quaternaire ou un groupe alkylsulfonate,

et Ar représente un groupe cyclique condensé comprenant des groupes cycliques aromatiques ou hétérocycliques;

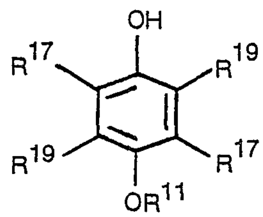

(7)

ou

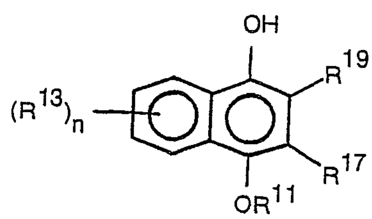

(8)

où:

$R^{19}$ représente un groupe aryle ou avec $R^{17}$ représente les atomes nécessaires pour réaliser un cycle condensé comprenant des groupes cycliques aromatiques ou hétérocycliques;

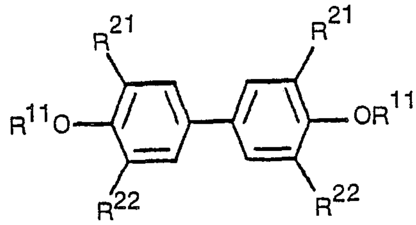

(9)

ou

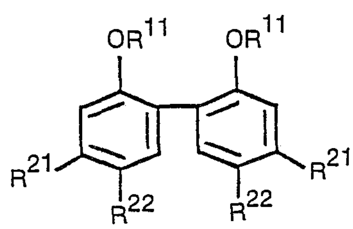

(10)

où:

$R^{21}$ représente un groupe alkyle, un groupe alcoxy, un groupe aryle ou un groupe dialkylamino, et

$R^{22}$ représente un groupe alkyle, un groupe aryle, un groupe alcoxy ou un groupe dialkylamino, ou avec $R^{21}$ représente les atomes nécessaires pour former un groupe cyclique alicyclique, oxyméthylène ou aromatique;

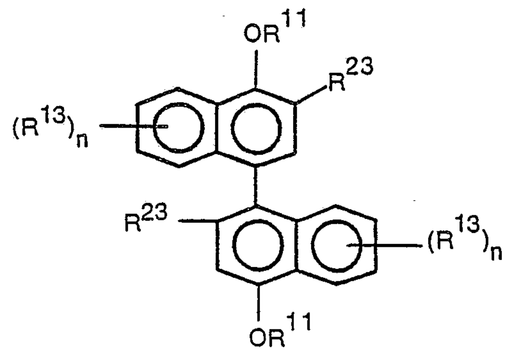

(11)

ou

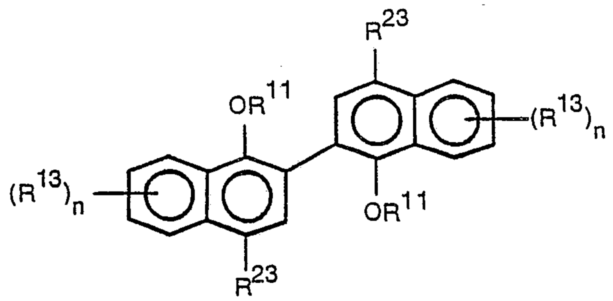

(12)

où:

$R^{23}$ représente un atome d'hydrogène ou un groupe alkyle, un groupe arylalkyle, un groupe alcoxy, un groupe aryloxy, un groupe alkylaryloxy, un groupe aryle, un groupe alkylaryle, un groupe alcoxyaryle, un groupe hydroxyaryle, un groupe aminoaryle, un groupe alkylaminoaryle, un groupe dialkylaminoaryle, un groupe carbalcoxy, un groupe carbaryloxy, un groupe carbamido, un groupe alkylamino, un groupe arylamino, un groupe diarylamino ou un groupe N-hétérocyclique et

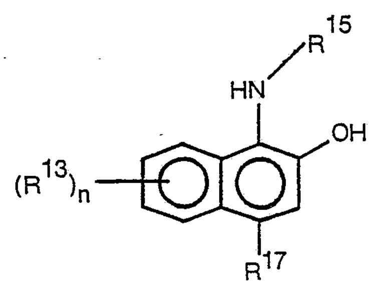

(13)

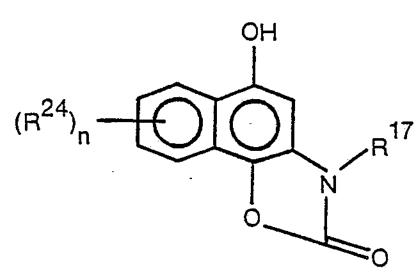

(14)

où:

$R^{24}$ représente un atome d'hydrogène, un groupe alkyle, un groupe alcoxy, un groupe hydroxyamino, un groupe alkylamino, un groupe dialkylamino, un groupe N-hétérocyclique ou un groupe aryle ou forme un groupe cyclique aromatique ou hétérocyclique condensé,

où dans toutes les formules ci-dessus $R^{11}$ est un hydrogène ou un groupe labile en conditions alcalines,

$R^{12}$ est un groupe de lestage ou les atomes nécessaires pour former un groupe cyclique hétérocyclique,

$R^{13}$ est choisi dans le groupe constitué par un hydrogène, les groupes alkyles, les groupes aryles, un hydroxy, les groupes alcoxy, les groupes aryloxy, les groupes amino, les groupes alkylamino, les groupes dialkylamino, les groupes arylamino, les groupes diarylamino, un carboxy, les groupes carbalcoxy, les groupes carbamido, un acide sulfonique, un sulfonate, les groupes arylsulfonyles, les groupes sulfalcoxy, les groupes sulfamido, un halogénure,

n est un entier choisi parmi 0, 1, 2, 3 et 4, et les groupes

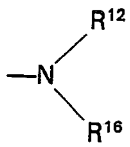

et

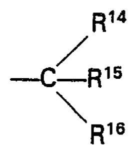

peuvent être situés uniquement dans les positions 2 et 4.

14. Un procédé de développement photographique dans lequel une pellicule photographique à halogénure d'argent exposée est développée dans un environnement alcalin en présence d'un ou plusieurs agents révélateurs formant une matière colorante répondant à la formule générale:

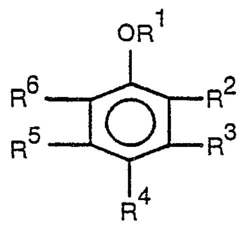

dans laquelle $R^1$ représente un atome d'hydrogène ou un groupe hydrolysable, et $R^2$ et $R^6$ sont indépendamment choisis parmi un hydrogène, un halogène, un groupe alkyle, un groupe aryle, un groupe alcoxy, un groupe aryloxy ou un groupe amino, un groupe hydroxy, un groupe thiol ou un groupe thioéther, ou deux ou plusieurs groupes adjacents choisis parmi $R^2$ à $R^6$ peuvent représenter les atomes nécessaires pour réaliser un ou plusieurs groupes cycliques carbocycliques ou hétérocycliques, et au moins deux des composants suivants:

(1) un solvant organique polaire miscible à l'eau,

(2) au moins une alcanolamine, une alcanolamine substituée et un acide monoamino-carboxylique pouvant être substitué, et

(3) un composé azoté choisi dans le groupe constitué d'une hydroxylamine, d'une hydroxylamine substituée, d'un acide arylhydroxamique ou d'un acide alkylhydoxamique pouvant chacun être substitué.

15. Un procédé comme revendique dans la revendication 14, dans lequel la pellicule est développée en présence d'un agent révélateur en noir et blanc.

16. Une émulsion photographique comprenant un colloïde hydrophile dans lequel sont dispersées des gouttelettes huileuses contenant des grains d'halogénure d'argent photographique et un révélateur formant une matière colorante de formule:

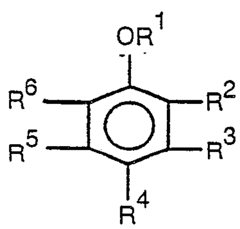

dans laquelle $R^1$ représente un atome d'hydrogène ou un groupe hydrolysable,

chacun de $R^2$ et $R^6$ est indépendamment choisi parmi un hydrogène, un halogène, un groupe alkyle, un groupe aryle, un groupe alcoxy, un groupe aryloxy ou un groupe amino, un groupe hydroxy, un groupe thiol ou un groupe thioéther, ou deux ou plusieurs groupes adjacents choisis parmi $R^2$ à $R^6$ peuvent représenter les atomes nécessaires pour réaliser un ou plusieurs groupes cycliques carbocycliques ou hétérocycliques, et au moins deux des composants suivants:

(1) un solvant organique polaire miscible à l'eau,

(2) au moins une alcanolamine, une alcanolamine substituée et un acide monoamino-carboxylique pouvant être substitué, et

(3) un composé azoté choisi dans la classe des hydroxylamines, des hydroxylamines substituées, des acides arylhydroxamiques et des acides alkylhydroxamiques pouvant être substitués.

17. L'émulsion photographique de la revendication 16 dans laquelle ledit révélateur formant une matière colorante répond à l'une quelconque des formules (3) à (14) suivantes:

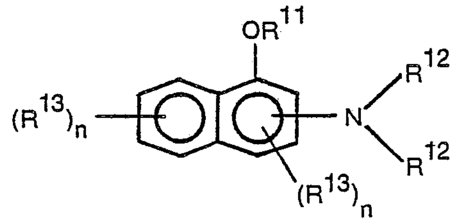

(3)

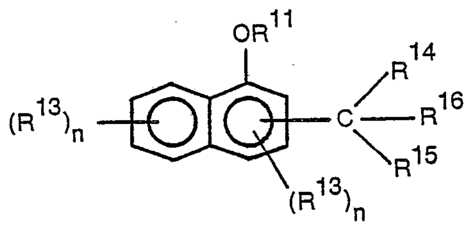

(4)

où

$R^{14}$ représente un groupe alkyle ou un hydrogène,

$R^{15}$ représente un atome d'hydrogène, un groupe alkyle ou un groupe aromatique, et

$R^{16}$ représente un groupe aromatique, ledit groupe étant capable d'activer l'hydrogène méthinique de l'agent révélateur lorsque $R^{14}$ ou $R^{15}$ représente un hydrogène, ou

$R^{16}$ avec $R^{15}$ et leur atome de carbone de liaison représentent les atomes nécessaires pour réaliser un groupe cyclique carbocyclique ou hétérocyclique qui est condensé ou un à un ou plusieurs cycles aromatiques autres que le noyau naphtalène, le groupe $—CR^{14}R^{15}R^{16}$ contenant au moins un cycle aromatique;

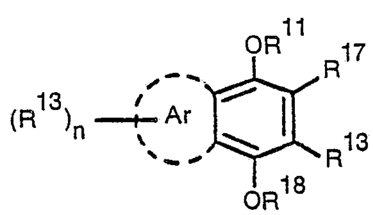

(5)

ou

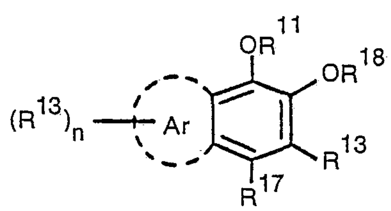

(6)

ou:

$R^{17}$ représente un hydrogène, un groupe alkyle, un groupe aryle, un groupe alkylaryle, un groupe alcoxyaryle, un groupe hydroxyaryle, un groupe aminoaryle, un groupe dialkylaminoaryle ou forme un cycle furanne avec le groupe $\alpha$-hydroxy,

$R^{18}$ représente un hydrogène, un groupe alkyle, un groupe arylalkyle, un groupe alcoxyalkyle, un groupe aminoalkyle, un groupe alkylammonium quaternaire ou un groupe alkylsulfonate,

et Ar représente un groupe cyclique condensé comprenant des groupes cycliques aromatiques ou hétérocycliques;

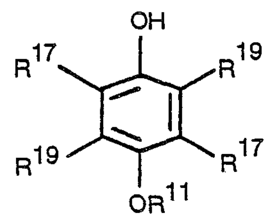

(7)

ou

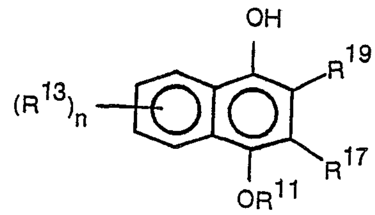

(8)

où:

$R^{19}$ représente un groupe aryle ou avec $R^{17}$ représente les atomes nécessaires pour réaliser un cycle condensé comprenant des groupes cycliques aromatiques ou hétérocycliques;

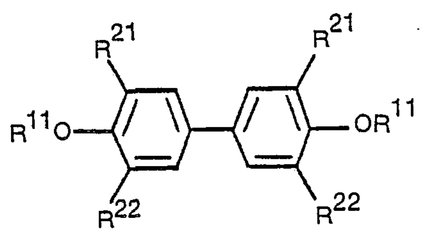

(9)

ou

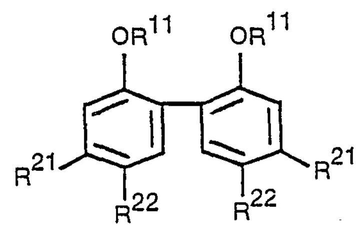

(10)

où:

$R^{21}$ représente un groupe alkyle, un groupe alcoxy, un groupe aryle ou un groupe dialkylamino, et

$R^{22}$ représente un groupe alkyle, un groupe aryle, un groupe alcoxy ou un groupe dialkylamino, ou avec $R^{21}$ représente les atomes nécessaires pour former un groupe cyclique alicyclique, oxyméthylène ou aromatique;

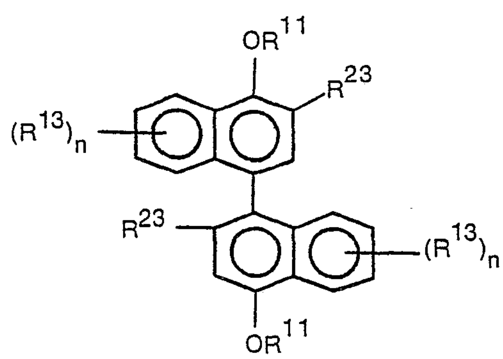

(11)

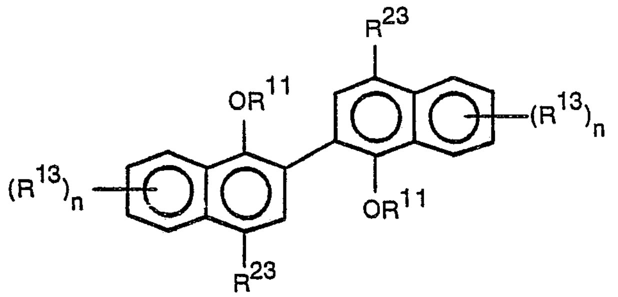

(12)

où:

$R^{23}$ représente un atome d'hydrogène ou un groupe alkyle, un groupe arylalkyle, un groupe alcoxy, un groupe aryloxy, un groupe alkylaryloxy, un groupe aryle, un groupe alkylaryle, un groupe alcoxyaryle, un groupe hydroxyaryle, un groupe aminoaryle, un groupe alkylaminoaryle, un groupe dialkylaminoaryle, un groupe carbalcoxy, un groupe carbaryloxy, un groupe carbamido, un groupe alkylamino, un groupe arylamino, un groupe diarylamino ou un groupe N-hétérocyclique et

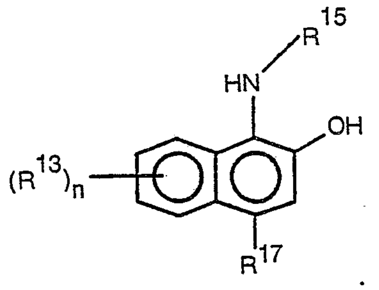

(13)

ou

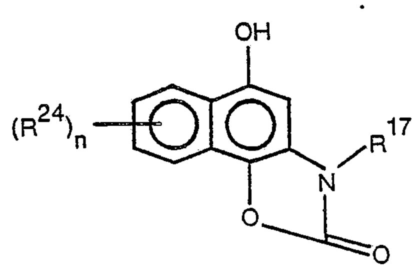

(14)

où:

$R^{24}$ représente un atome d'hydrogène, un groupe alkyle, un groupe alcoxy, un groupe hydroxyamino, un groupe alkylamino, un groupe dialkylamino, un groupe N-hétérocyclique ou un groupe aryle ou forme un groupe cyclique aromatique ou hétérocyclique condensé,

où dans toutes les formules ci-dessus $R^{11}$ est un hydrogène ou un groupe labile en conditions alcalines,

$R^{12}$ est un groupe de lestage ou les atomes nécessaires pour former un groupe cyclique hétérocyclique,

$R^{13}$ est choisi dans le groupe constitué par un hydrogène, les groupes alkyles, les groupes aryles, un hydroxy, les groupes alcoxy, les groupes aryloxy, les groupes amino, les groupes alkylamino, les groupes dialkylamino, les groupes arylamino, les groupes diacylamino, un carboxy, les groupes carbalcoxy, les groupes carbamido, un acide sulfonique, un sulfonate, les groupes arylsulfonyles, les groupes sulfalcoxy, les groupes sulfamido, un halogénure,

n est un entier choisi parmi 0, 1, 2, 3 et 4, et les groupes

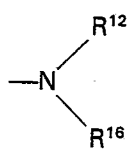

et

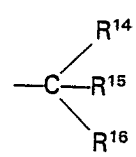

peuvent être situés uniquement dans les positions 2 et 4.

18. L'émulsion des revendications 16 ou 17 dans laquelle ledit révélateur formant une matière colorante est présent dans un rapport d'au moins 2,0 moles de révélateur par mole d'halogénure d'argent.